(19)

Europäisches
Patentamt
European
Patent Office
Office européen
des brevets

(11) **EP 3 705 891 A1**

(12) **EUROPEAN PATENT APPLICATION**

(43) Date of publication:
**09.09.2020   Bulletin 2020/37**

(51) Int Cl.:
*G01N 33/50* *(2006.01)*

(21) Application number: **19305263.6**

(22) Date of filing: **06.03.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Novagray
34960 Montpellier Cedex 02 (FR)**

• **Institut Régional du Cancer de Montpellier
34090 Montpellier (FR)**
• **Université de Montpellier
34090 Montpellier (FR)**

(72) Inventor: **The designation of the inventor has not
yet been filed**

(74) Representative: **Flesselles, Bruno F.G.
BF IP
36 rue Jean de la Fontaine
75016 Paris (FR)**

(54) **IN VITRO METHOD FOR ASSESSING THE RISK OF PROSTATE SIDE EFFECT AFTER
TREATMENT BY IONIZING RADIATION**

(57)    The present invention relates to an *in vitro* method for assessing the risk of developing side effects after ionizing radiation treatment in a prostate cancer subject, comprising the steps of
a) determining the presence or absence of urinary toxicity in the patient prior to application of ionizing radiation,
b) determining the stage of the tumor,
c) optionally determining of at least one other clinical parameter, disease parameter or ionizing radiation treatment parameter from the patient, and
d) combining a value associated with the presence of urinary toxicity determined in a), a value associated with the stage of the tumor in b), and a value association with the at least one other clinical parameter, disease parameter or ionizing radiation treatment parameter determined in c) in a mathematical function to obtain an end-value

EP 3 705 891 A1

## Description

### FIELD OF INVENTION

The present invention relates to the prediction of a risk of developing side effects after treatment by ionizing radiation (TIR) in a prostate cancer patient. The present invention thus provides an *in vitro* method and a calculator for assessing the risk of developing side effects after TIR.

### BACKGROUND OF INVENTION

Treatment by ionizing radiation (TIR) is one of the leading treatment modalities in oncology, and over 50% of patients diagnosed with cancer undergo TIR during their course of treatment. Also TIR is primarily a local treatment, patients are exposed to a risk of toxicities in the treatment field and surrounding tissues, which may develop acutely (*i.e.*, in the first 3 months) or late (*i.e.*, more than 3 months following RT). Severe acute toxicities may have late consequences as recovering may be incomplete. In addition, late toxicities may occur over time and often persist with significant negative impact on quality of life among cancer survivors.

A number of factors are known to increase the risk of radiation-induced toxicity, including individual radiosensitivity (Azria, Betz et al. 2012). While toxicity risks for populations of patients are known, the determination of an individual's normal tissue radiosensitivity is seldom possible before treatment. Therefore, current practice standards commonly prescribe radiation dose according to clinical scenarios from standard recommendations, without regard to the genotype or phenotype of the individual being irradiated.

In that context, a radiosensitivity prognostic test was developed, based on flow cytometric assessment of RILA (radiation-induced T-lymphocytes apoptosis). RILA corresponds to the difference of percentage (%) between the rate of radiation-induced T lymphocytes apoptosis after an irradiation of a T lymphocyte containing sample of the subject (preferably a blood sample), with X-Rays (e.g., 8 Gy X-Rays) and the rate of lymphocytes apoptosis without any irradiation (0 Gy). Preferably, T lymphocytes are CD8 lymphocytes. T lymphocytes can also be CD4 lymphocytes. In summary, the RILA test consists in irradiating lymphocytes of the patient and detecting the apoptosis rate. In particular a dose of 8Gy is administered to lymphocytes (preferably a 1Gy/mn) on lymphocytes of a blood sample (between 24 and 48 hours after harvest) and apoptosis rate is determined after 48 hours of sample incubation after irradiation. The lymphocytes for which apoptosis rate is evaluated are preferably CD8 lymphocytes. Apoptosis rate is evaluated by methods known in the art, and in particular using flow cytometry. This prognostic test was described as having a clear potential for selecting individuals likely to display an increased probability of toxicity to TIR (Ozsahin, Crompton et al. 2005). Nevertheless, the prediction of this radiosensitivity assay based on RILA exhibits some limitations in terms of sensitivity and reliability. Indeed, taken alone, RILA is not capable of exhaustive prediction in terms of sensitivity and specificity.

Therefore, there is an important need to develop a method of prognostic predicting the risk of developing radiation-induced side effects, such as prostate side effects after TIR, having good sensitivity and reliability. Furthermore, the RILA process may be difficult to perform as it requires time (about four days) and specific devices for irradiating the lymphocytes.

Foro et al (Int J Radiat Oncol Biol Phys. 2014 Apr 1;88(5):1057-63) assess the correlation between radiation-induced apoptosis of T lymphocytes (RILA) and chronic toxicity in prostate cancer patients. They propose to use univariate or multivariate Cox proportional hazard models. Tables 3 and 4 present various parameters proposed by the authors. These parameters don't include the presence of urinary toxicity before treatment.

De Langhe et al. (2013, Radiotherapy and Oncology, Volume 106, S350) present an abstract pertaining to the prediction of occurrence of radiation-induced genitourinary (GU) symptoms such as nocturia or hematuria. They combine multiple factors including clinical, treatment, dosimetric and genetic parameters. Presence of pre-treatment urinary disorders or RILA are not among these parameters.

US 2016/008629 discloses a system and method for detecting or predicting toxicity induced by radiation therapy of prostate cancer, in particular by determining polypeptide biomarkers present in a urine sample. It proposes combine the biomarker concentrations with data from, for example, medical imaging, disease and/or patient specific information, such as genetic data, tumor grade and stage, and co-morbidities to obtain even more reliable predictions. However, no such combination is provided, and RILA or presence of pre-treatment urinary disorders are not cited.

WO2018/041960 discloses a mathematical function combining RILA and other factors (such as tabagism or presence of concomitant hormonotherapy) to detect the occurrence of late effects in breast cancer patient treated with radiotherapy. The factors combined with RILA are adapted for breast cancer but not for prostate cancer.

### SUMMARY

The invention now provides a new prognosis method for predicting the probability or risk of developing radiation-induced side effects, in particular a prostate side effect (PSE), wherein the tumor stage (according to the TNM Classification of Malignant Tumors) is combined with the presence of pre-treatment urinary toxicity, and optionally with other clinical parameters, disease parameters and/or ionizing radiation treatment parameters, and in particular with neoadjuvant, concomitant and/or adjuvant hormonotherapy, with an improved global evaluation of the risk of developing a PSE for a patient. This method makes it possible to make a prognosis of the occurrence of such PSE, in particular within two years (24 months) after the ionizing radiation treatment. Full advantages of the method are to be found when it is performed before the start of treatment of a patient with prostate cancer, as it provides the physician with valuable information as to the nature of the treatment that can be proposed to the patient, depending on the patient's risk of having some PSE.

The inventors show herein that it is possible to develop new prognosis tests that don't include RILA as a variable, and that combine the presence (and optionally grade) of utinary disorders and of the stumor stage, optionally with other parameters. These two parameters are preferably combined with at least one other parameter, in particular with exactly one other parameter, or with two other parameters, or with three other parameters, or with four other parameters. It is also possible to use further parameters. It is believed that it is the first time that a link between presence of urinary disorders prior to treatment (alone or in combination with tumor stage) and susceptibility to the occurrence of side effects has been shown in prostate cancer patients.

The present invention relates to an *in vitro* method for assessing the risk of developing side effects after ionizing radiation in a patient suffering from prostate cancer, comprising:

  a) determining the presence or absence, of urinary toxicity in the patient prior to application of ionizing radiation,
  b) determining the stage of the patient's tumor,
  c) optionally determining of at least one other clinical parameter, disease parameter or ionizing radiation treatment parameter from the patient, and
  d) combining a value associated with the presence of urinary toxicity determined in a), a value associated with the stage of the tumor in b), and a value association with the at least one other clinical parameter, disease parameter or ionizing radiation treatment parameter determined in c) in a mathematical function to obtain an end-value.

Preferably, the tumor stage determined in b) is the T stage.

Preferably, a) also comprises the determination of the grade of the urinary toxicity if such toxicity is present in the patient.

Such end-value can later be compared to a reference value, in order to determine the risk, for the patient to developing side effects after a ratio-therapy treatment, or a treatment with ionizing radiation. This method thus also makes it possible to determine if the patient a no, or a low risk to develop such side effects.

This method is preferably performed before starting the treatment of the patient. In this case, if a ionizing radiation treatment parameter is used in the method, the ionizing radiation treatment parameter is the one that is envisaged for the patient.

In one embodiment, the method comprises measuring or determining at least one other clinical marker than urinary toxicity and tumor stage, preferably selected from the group consisting of the presence or absence of concomitant and/or adjuvant hormonotherapy, the tobacco smoking habit, the presence of gastrointestinal toxicity, the dosage of PSA (Prostate Specific Antigen), the presence or absence of lymph node dissection and the presence of at least one comorbidity (in particular diabetes or intake of anticoagulants). In this case, a value associated with said at least one other clinical marker is also used and combined in the function of c). If more than one other clinical marker is also measured or determined, the values associated with each of the measured or determined clinical markers are used in c).

In one embodiment, the age of the patient is also taken into consideration and, combined in the function of c).

In one embodiment, the method comprises using at least one ionizing radiation treatment parameter, preferably CTV, CTV1 and/or CTV2 in b) for combination in c).

In one embodiment, the mathematical function of c) is a multivariate analysis using a binary logistic regression, a multiple linear regression or any time dependent regression a binary logistic regression, a multiple linear regression or any time dependent regression.

In one embodiment, said mathematical function is a Cox proportional hazard regression model.

In one embodiment, the method of the invention further comprises a step of comparing the end-value with a reference end-value, thereby determining if the subject present a high or low risk to develop radiation-induced side-effects.

In one embodiment, said radiation-induced side effect is a side effect occurring during ionizing radiation and/or during the follow-up after ionizing radiation, such as, for example, 1 month, 3, 6, 12, 18, 24, 30 or 36 months after the end of the ionizing radiation. In particular, the method is particularly adapted to determine the risk of occurence of a PSE (radiation-induced side effect) in the 24 months following the treatment.

In one embodiment, said radiation-induced side effect is selected from urinary toxicities, gastrointestinal toxicities, sexual toxicities and pelvic neuropathy.

In one embodiment, said method is computerized.

The present invention further relates to a microprocessor comprising a software for implementing the *in vitro* method as described hereinabove.

The present invention also relates to a method for treating a patient having prostate cancer, comprising:

a) performing a method as disclosed above, and

b) treating the patient with ionizing radiation, if the end result indicates that the subject has no or a low risk of developing side effects after ionizing radiation,

c) providing another treatment or adapting the ionizing radiation treatment if the patient doesn't have no or a low risk of developing side effects after ionizing radiation.

In particular, the ionizing radiation treatment can be adapted taking into account the risk of developing PSE together with the risk of tumor recurrence.

The invention also relates to a method for obtaining a mathematical function that can be used in an in vitro non-invasive diagnosis test for assessing the risk of developing side effects after ionizing radiation in a subject suffering from prostate cancer, comprising:

a) classifying subjects of a cohort of patients into different groups according to the presence of side effects after ionizing radiation treatment

b) determining the presence or absence of urinary toxicity prior to ionizing radiation treatment,

c) determining the stage of the patient's tumor according to the TNM staging system

d) optionally determining the presence of at least one other marker selected from the group consisting in the presence or absence of concomitant and/or adjuvant hormonotherapy, the tobacco smoking habit, the presence or absence of gastrointestinal toxicity, the dosage of PSA (Prostate Specific Antigen), the presence of at least one comorbidity (in particular diabetes), the presence or absence of lymph node dissection, and the age of the patient, before ionizing radiation treatment

e) identifying by unidimensional analysis, the markers measured in step d) for which the measured value differs significantly between the group of subjects in which side effects after ionizing radiation have developed and the group of subjects in which side effects after ionizing radiation have not occurred, thereby obtaining independent factors

f) obtaining the mathematical function by combination of the factor measured in b), the marker determined in c) and the identified independent factors identified in e).

**DEFINITIONS**

In the present invention, the following terms have the following meanings:

- The terms "**a**" and "**an**" refer to one or to more than one (*i.e.*, to at least one) of the grammatical object of the article. By way of example, "an element" means one element or more than one element.
- The term "**about**" when referring to a measurable value such as an amount, a temporal duration, and the like, is meant to encompass variations of $\pm20\%$ or in some instances $\pm10\%$, or in some instances $\pm5\%$, or in some instances $\pm1\%$, or in some instances $\pm0.1\%$ from the specified value, as such variations are appropriate to perform the disclosed methods.
- The term "**biochemical marker**" refers to a variable that may be measured in a sample from the subject, said sample being preferably a blood sample.
- The term "**Cox regression**" refers to a usual statistical model for time-to-event analysis (Cox, et al. 1984). Apart from a classification algorithm which directly deals with binary or multi-class outcomes, Cox regression defines a semi-parametric model to directly relate the predictive variables with the real outcome, which may be, for example, a survival time (*e.g.*, in months or years) or a time without occurrence of side effects or recurrence of a disease. Multivariate Cox function is considered as the best hazard function in terms of discrimination for time-to-event endpoint to combine independent parameters.
- The term "**encoding**" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*e.g.*, rRNA, tRNA and mRNA) or a defined sequence of amino acids and the biological properties resulting therefrom. Thus, a gene, cDNA, or RNA, encodes a protein if transcription and translation of mRNA corresponding to that gene, or cDNA, produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

- The term "**expression**" refers to the transcription and/or translation of a particular nucleotide sequence driven by a promoter.

- The term "**instructional material**" includes a publication, a recording, a diagram, or any other medium of expression which can be used to communicate the usefulness of the kit of the invention. The instructional material of the kit of the invention may, for example, be affixed to a container which contains the reagents for implementing the method of the invention or be shipped together with a container which contains the reagents for implementing the method of the invention. Alternatively, the instructional material may be shipped separately from the container with the intention that the instructional material be used cooperatively by the recipient.

- The term "***in vitro* method**" refers to a method comprising steps performed *in vitro* (*e.g.*, measure of the PSA level) or *ex-vivo* (*e.g.*, multivariate cox regression model obtained with clinical parameters or disease parameters or ionizing radiation treatment parameters previously evaluated on patients).

- The term "**non-invasive**", when referring to a method according to the present invention, means that the method of the invention does not comprise obtaining a tissue sample from the body of a subject. In one embodiment, a blood sample is not considered as a tissue sample.

- "**ROC**" In statistics, a receiver operating characteristic (ROC), or ROC curve, is a graphical plot that illustrates the performance of a binary classifier system as its discrimination threshold is varied. The curve is created by plotting the sensitivity against the specificity (usually 1- specificity) at successive values from 0 to 1. "**AUROC**" stands for area under the ROC curve, and is an indicator of the accuracy of a prognostic or diagnostic test. ROC curve and AUROC are well-known in the field of statistics.

- The term "**sensitivity of a method of prognosis**" refers to the proportion of patients with a risk to develop side-effect that are correctly identified as such using a method of prognosis.

- The term "**specificity of a method of prognosis**" refers to a measure of the proportion of patients without risk to develop side-effect that are correctly identified as such using a method of prognosis.

- The term "**side effect**" (or adverse event) refers to an unfavorable and unintended sign (including an abnormal laboratory finding), symptom or disease temporally associated with the use of a medical treatment. In particular, a radiation-induced side effect is a side effect induced in a subject by an ionizing radiation treatment. Severity of side effects may be defined according to the Common Terminology Criteria for Adverse Events (CTCAE, *e.g.*, CTCAE v3.0 or CTCAE v4.0). According to the CTCAE, globally the following grades of side effects may be distinguished: Grade 1: mild side effect, Grade 2: moderate side effect, Grade 3: severe side effect, Grade 4: life threatening or disabling side effect and Grade 5: death related to side effect, but specifically defined for each symptom. In one embodiment of the invention, the side effect is at least a Grade 2 side effect. In one embodiment, the side effect is a Grade 2, 3, 4 or 5 side-effect, preferably a Grade 2, 3 or 4 side-effect. In the present description, reference is made to the CTCAE V3.0, published by the U.S.DEPARTMENT OF HEALTH AND HUMAN SERVICES (National Institutes of Health, National Cancer Institut, Cancer Therapy Evaluation Program), March 31, 2003 and published on August 9, 2006.

- The term "**subject**" or "**patient**" is intended to include any living organisms (*e.g.*, mammals, preferably humans). In one embodiment, a subject is a warm-blooded animal, preferably a human, who/which is awaiting the receipt of, or is receiving medical care or was/is/will be the object of a medical procedure, or is monitored for the development of the targeted disease or condition. In one embodiment, the subject is an adult (for example a subject above the age of 18). In another embodiment, the subject is a child (for example a subject below the age of 18).

- The terms "**treat**", "**treatment**" and "**treating**" refer to the reduction or amelioration of the progression, severity and/or duration of a targeted cancer, or to the amelioration of one or more symptoms (preferably, one or more discernible symptoms) of a targeted cancer, wherein said amelioration results from the administration of one or more therapies. In one embodiment the terms "treat", "treatment" and "treating" refer to the inhibition of the progression of a targeted cancer, either physically by, *e.g.*, stabilization of at least one discernible symptom, physiologically by, *e.g.*, stabilization of a physical parameter, or both. In other embodiments the terms "treat", "treatment" and "treating" refer to the reduction or amelioration of the progression, severity and/or duration of a targeted cancer, or to the amelioration of one or more symptoms of a targeted cancer. A subject is successfully "treated" for a cancer if, after receiving a therapeutically effective amount of a therapeutic agent, the subject shows observable and/or measurable reduction in the number of pathogenic cells or reduction in the percent of total cells that are pathogenic; relief to some extent of one or more of the symptoms associated with the specific cancer; reduced morbidity and mortality, and/or improvement in quality of life issues. The above parameters for assessing successful treatment and improvement in the condition are readily measurable by routine procedures familiar to a physician.

## DETAILED DESCRIPTION

The present invention first relates to an *in vitro* method for assessing the risk of developing side effects after ionizing radiation in a subject having prostate cancer, wherein said method comprises the steps of:

> a) determining the presence or absence a urinary toxicity in the subject;
> b) determining the stage of the patient's tumor, preferably the T stage,
> c) Optionally measuring or determining for the subject at least one other of:
>
>> ∘ at least one clinical parameter; and/or
>> ∘ at least one disease parameter; and/or
>> ∘ at least one ionizing radiation treatment parameter, and
>
> d) combining
>
>> ∘ a value associated with the presence or absence of urinary toxicity
>> ∘ a value associated with the stage of the tumor, and
>> ∘ the values associated with any orther clinical, disease or ionizing radiation treatment parameter of c), if relevant,

in a mathematical function to obtain an end-value.

In one embodiment, the method of the invention comprises measuring or determining at least one other clinical parameter. In one embodiment, the method of the invention comprises measuring or determining at least one disease parameter. In one embodiment, the method of the invention comprises measuring or determining at least one ionizing radiation treatment parameter. In one embodiment, the method of the invention comprises measuring or determining at least one other clinical parameter and at least one disease parameter. In one embodiment, the method of the invention comprises measuring or determining at least one other clinical parameter and at least one ionizing radiation treatment parameter. In one embodiment, the method of the invention comprises measuring or determining at least one disease parameter and at least one ionizing radiation treatment parameter. In one embodiment, the method of the invention comprises measuring or determining at least one other clinical parameter, at least one disease parameter and at least one ionizing radiation treatment parameter. In one embodiment, the method of the invention further comprise using the age of the patient in the mathematical function.

The method of the invention comprises combining the different values measured or associated with the markers of a) to c) in a mathematical function to obtain an end-value.

In one embodiment, the method of the invention is non-invasive.

In one embodiment, the subject is a human.

In one embodiment, the subject is treated by ionizing radiation for a prostate cancer. Therefore, the subject is preferably a male. In another embodiment, the subject is not yet treated for prostate cancer when the method if applied.

In one embodiment, said prostate cancer is castrate-resistant prostate cancer, metastatic castrate-resistant prostate cancer, recurrent prostate cancer, unresectable prostate cancer, advanced prostate cancer, or locally advanced prostate cancer.

As used herein, advanced cancer is cancer that has spread to other places in the body and usually cannot be cured or controlled with treatment. In particular, locally advanced cancer is cancer that has spread from where it started to nearby tissue or lymph nodes. Unresectable cancer is a cancer that is unable to be removed with surgery. Recurrent cancer is cancer that has recurred (come back). The cancer may come back to the same place as the original (primary) tumor or to another place in the body. Metastatic cancer is cancer that has spread from the place where it first started to another place in the body. A tumor formed by metastatic cancer cells may be called a metastatic tumor or a metastasis. The metastatic tumor contains cells that are like those in the original (primary) tumor. Castrate-resistant prostate cancer is prostate cancer that keeps growing even when the amount of testosterone in the body is reduced to very low levels. Many early-stage prostate cancers need normal levels of testosterone to grow, but castrate-resistant prostate cancers do not.

The American Joint Committee on Cancer (AJCC) classifies prostate cancer according to a tumor, nodes, metastasis (TNM) classification system for prostate cancer. TNM Stage III includes the criterion that 'Tumor extends through the prostate capsule'. Hence, Stage III prostate cancer is broadly equivalent to locally advanced prostate cancer. TNM Stage IV disease meets the primary tumor criterion that the "tumor is fixed or invades adjacent structures other than seminal vesicles such as external sphincter, rectum, bladder, levator ani, and/or pelvic wall". Hence, stage IV prostate cancer is broadly equivalent to advanced prostate cancer. TNM Stage IV disease is also defined as any occurrence of metastasis in regional lymph node(s) or any occurrence of distant metastasis. Hence, stage IV prostate cancer is broadly equivalent to metastatic prostate cancer. TNM was developed and is maintained by the Union for International Cancer Control (UICC).

In one embodiment, prostate cancer is TNM stage I prostate cancer, including Ia, Ib, Ic..

In another embodiment, prostate cancer is TNM stage II prostate cancer.

In another embodiment, prostate cancer is TNM stage III prostate cancer.

In another embodiment, prostate cancer is TNM stage IV prostate cancer.

In one embodiment, said sample is a T cell containing sample.

Examples of T cell containing samples include, but are not limited to, whole blood samples and samples recovered from bone marrow, lymph node tissue, cord blood, thymus tissue, tissue from a site of infection, ascites, pleural effusion, spleen tissue, and tumors. In one embodiment of the present invention, the T cell containing sample can be obtained from a unit of blood collected from a subject using any number of techniques known to the skilled artisan, such as, for example, by leukapheresis.

In one embodiment, said sample is a bodily fluid sample, such as, for example, a blood, plasma, serum, lymph, urine, cerebrospinal fluid or sweat sample.

In one embodiment, said sample is a blood sample.

In one embodiment, the sample is recovered prior to the implementation of the method of the invention, *i.e.*, the step of recovering the sample is not part of the method of the invention.

According to the invention, the subject is, was or will be treated by ionizing radiation.

It is preferred when the subject is planned to be treated by ionizing radiation, and the method of the invention is implemented before the beginning of the TIR.

The term "ionizing radiation", as used herein, refers to a treatment involving the use of high-energy radiation such as, for example, X-rays, gamma rays, electron beams or protons, to kill or damage cancer cells and stop them from growing and multiplying.

In one embodiment, the ionizing radiation involves the use of X-rays.

In one embodiment, the ionizing radiation involves the use of brachytherapy or brachytherapy boost.

The side effects are side effects induced by ionizing radiation in the pelvic area.

Examples of radiation-induced side effects include, but are not limited to, genitourinary (such as, for example, urinary and sexual toxicities), gastrointestinal and neurologic toxicities.

Examples of radiation-induced urinary toxicities include, but are not limited to, dysuria, pollakiuria, abnormal (*e.g.*, increased) urinary frequency, decreased urine stream, burning urination, bleedings, urinary incontinence, mictional or urinary impetuosity, nocturia, hematuria, and urinary pain.

Examples of radiation-induced gastrointestinal toxicities include, but are not limited to, diarrhea, constipation, rectal syndrome, functional proctitis and/or anusitis, physical proctitis and/or anusitis, radiation proctitis, ano-proctitis, bleeding (in particular rectal bleeding or anal bleeding), and gastrointestinal pain (in particular rectal or anal pain).

Examples of radiation-induced sexual toxicities include, but are not limited to, sexual impotence, erectile dysfunction, and ejaculation dysfunction.

Another example of radiation-induced side effects is pelvic neuropathy.

In one embodiment, the method of the invention aims at predicting the risk of developing side effects during ionizing radiation treatment and a period of about 1, 3, 6, 12, 18, 24, 30 or 36 months after TIR. It is particularly adapated to predicting the risk of developing side effects within 24 months after the TIR.

In one embodiment, the *in vitro* method of the invention aims at predicting the risk of developing acute side effects, *i.e.*, side effects occurring during TIR or less than about 1 week, 2 weeks, 3 weeks or 4 weeks after TIR, or less than about 1, 2 or 3 months after TIR. Such acute side effects of the pelvic area may be referred to as Prostate Side Effect (or PSE).

In another embodiment, the *in vitro* method of the invention aims at predicting the risk of developing late side effects, *i.e.*, side effects occurring at least about 3 months after ionizing radiation treatment, such as, for example, between about 3 months and about 6 months after ionizing radiation treatment, between about 3 months and about 12 months after ionizing radiation treatment, between about 3 months and about 18 months after ionizing radiation treatment, between about 3 months and about 2 years after ionizing radiation treatment, between about 3 months and about 30 months after ionizing radiation treatment, or between about 3 months and about 3 years after ionizing radiation treatment. In one embodiment, the late side effects occur about 4, 5, 6, 7, 8, 9, 10, 11, 12 months or more after ionizing radiation treatment, or 2 or 3 years or more after ionizing radiation treatment. In one embodiment, such late side effects of the pelvic area may be referred to as Prostate Side Effect (or PSE).

In one embodiment, the side-effects as listed hereinabove are at least Grade 2 side effects according to the CTCAE, in particular to the v3.0 CTCAE. In one embodiment, the side-effects as listed hereinabove are Grade 2, Grade 3, Grade 4 or Grade 5 side effects according to the CTCAE, in particular to the v3.0 CTCAE, preferably Grade 2, Grade 3, or Grade 4.

In one embodiment, said at least one other biochemical marker measured in step (a) is selected from the group comprising proteins of radiosensitivity and genes of radiosensitivity.

In one embodiment, at least one other biochemical marker is measured and used in the function. Said at least one other biochemical marker can be a protein of radiosensitivity, preferably selected from the group consisting of AK2 (adenylate kinase 2), HSPA8 (Heat shock cognate protein 71 kDa, also referred to as HSC70), ANX1 (Annexin 1), APEX1 (DNA-(apurinic or apyrimidinic site) lyase) and ID2 (mitochondrial isocitrate dehydrogenase 2, that may also be referred to as IDH2), fragments and combinations thereof.

In one embodiment, said at least one other biochemical marker is a combination of at least two proteins of radiosensitivity, of at least three proteins of radiosensitivity, of at least four proteins of radiosensitivity, or of five proteins of radiosensitivity, preferably selected from the group consisting of AK2, HSPA8, ANX1, APEX1 and ID2.

As used herein, a "protein of radiosensitivity" refers to a protein whose expression (either at the protein or RNA level) is indicative of the radiosensitivity of the subject.

Consequently, in one embodiment, measuring at least one other biochemical marker at step (a) corresponds to measuring a protein level of at least one protein of radiosensitivity in a sample from the subject or to measuring a nucleic acid encoding said protein in a sample from the subject. In one embodiment, in a first step, proteins and/or nucleic acids are isolated from a biological sample previously obtained from the subject. A method according to the invention may thus include protein or nucleic acid extraction, purification and characterization, using well known biochemistry methods.

The presence or level of said protein of radiosensitivity may be determined by methods well known in the art. Examples of such methods include, but are not limited to, a method based on immune-detection, a method based on western blot, a method based on mass spectrometry, a method based on chromatography, or a method based on flow cytometry, and a method for specific nucleic acid detection. Specific examples of *in vitro* methods for determining a protein level in a sample are well-known in the art, and include, but are not limited to, immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

In one embodiment, the presence and level of expression of proteins can be determined directly or be analyzed at the nucleic level by detecting, and preferably quantifying, protein-specific nucleic acids, and particularly mRNA (*i.e.*, assessing the transcription level of the protein). Methods for assessing the transcription level of a protein are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

In one embodiment, said at least one biochemical marker is a gene of radiosensitivity, preferably selected from the group consisting of TGFβ, SOD2, TNFα, and XRCC1.

As used herein, a "gene of radiosensitivity" refers to a gene whose expression (either at the protein or RNA level) is indicative of the radiosensitivity of the subject, or to a gene comprising at least one single nucleotide polymorphism (SNP) indicative of the radiosensitivity of the subject, or involved in the fibrosis pathway and ROS management.

Consequently, in one embodiment, measuring at least one other biochemical marker at step (a) corresponds to measuring an expression level of or determining the presence of a SNP in at least one gene of radiosensitivity in a sample from the subject. In this embodiment, the value associated with the presence (or absence) of the SNP will be 1 (or 0). Alternatively, if the expression level is measured, the measured value can be used in the formula.

The presence or level of expression of said gene of radiosensitivity may be determined by a usual method known from the person skilled in the art. A nonlimiting list of such methods is shown hereinabove.

In one embodiment, the method for determining presence or level of expression of a gene of radiosensitivity is as disclosed in Azria et al., 2008 and includes lymphocyte isolation, DNA extraction and amplification, and denaturating high-performance liquid chromatography or the Surveyor nuclease assay using a Transgenomic WAVE High Sensitivity Nuclei Acid Fragment Analysis System. PCR primers for the DNA amplicons encompassing the SNPs of interest disclosed above may designed using, for example, the genomic sequence obtained from the NCBI.

In one embodiment, the method of the invention comprises measuring at least one protein of radiosensitivity. In another embodiment, the method of the invention comprises measuring at least one gene of radiosensitivity. In another embodiment, the method of the invention comprises measuring at least one protein of radiosensitivity and at least one gene of radiosensitivity.

It is also interesting to use, as one other biochemical marker, the level of Prostate Specific Antigen (PSA) in the blood of the patient.

By "clinical parameter" it is meant any clinical parameter related to the subject and relevant to assess an increased risk of radiation-induced toxicity in said subject. One clinical parameter (presence of urinary toxicity) is determined according to the present disclosure.

Examples of clinical parameters include, but are not limited to, lymph node dissection, concomitant medical treatment (*e.g.*, neoadjuvant, concomitant and/or adjuvant hormonotherapy or anticoagulant therapy), age, presence or history of other diseases or conditions, preferably before ionizing radiation (which may also be referred to as the presence of at least one comorbidity, wherein said comorbidities may be selected, without limitation, from high blood pressure, diabetes, angina pectoris, heart failure, arteritis, chronic hepatitis, respiratory failure, sexual disorders, digestive disorders), and tobacco smoking.

In particular, it is determined that the patient has urinary toxicity (or urinary disorder) if the patient has at least one of dysuria (painful urination), urine color change (in particular hematuria), pollakiuria, bladder spasms, urinary frequency/urgency, urinary retention, urinary leak, incontinence, obstruction or stricture/stenosis (bladder, ureter or urethra), urinary tract infection (such as cystitis) and/or erectile dysfunction and when the trouble is graded 1 or more, according to the CTCAE v3.0 when relevant. Other signs of urinary toxicity may be used.

It is also possible to use, as the marker of urinary toxicity, only one of the above marker, in particular dysuria.

If the patient presents no urinary toxicity, the value associated with this presence of the clinical marker in the function is 0.

If the patient presents such urinary toxicity, the value associated with this presence of the clinical marker in the function depends on the value of the grade as determined in the CTCAE v3.0.

Alternatively, one can assess the value 1 in case of presence of urinary toxicity, whatever the grade of such.

The method also uses the staging of the tumor according to the TNM system. In particular, the T grade is determined.

Using the TNM system, the "T" plus a letter or number (0 to 4) is used to describe the size and location of the tumor. Some stages are also divided into smaller groups that help describe the tumor in even more detail.

The different T grades are the following: T0: There is no evidence of a tumor in the prostate; T1: The tumor cannot be felt during a digital rectal exam and is not seen during imaging tests. It may be found when surgery is done for another reason, usually for benign prostatic hyperplasia or an abnormal growth of noncancerous prostate cells. T1a: The tumor is in 5% or less of the prostate tissue removed during surgery. T1b: The tumor is in more than 5% of the prostate tissue removed during surgery. T1c: The tumor is found during a needle biopsy, usually because the patient has an elevated PSA level. T2: The tumor is found only in the prostate, not other parts of the body. It is large enough to be felt during a digital rectal exam. T2a: The tumor involves one-half of 1 lobe (part or side) of the prostate. T2b: The tumor involves more than one-half of 1 lobe of the prostate but not both lobes. T2c: The tumor has grown into both lobes of the prostate. T3: The tumor has grown through the prostate capsule on 1 side and into the tissue just outside the prostate. T3a: The tumor has grown through the prostate capsule either on 1 side or on both sides of the prostate, or it has spread to the neck of the bladder. This is also known as an extraprostatic extension (EPE). T3b: The tumor has grown into the seminal vesicle(s), the tube(s) that carry semen. T4: The tumor is fixed, or it is growing into nearby structures other than the seminal vesicles, such as the external sphincter, the part of the muscle layer that helps to control urination; the rectum; levator ani; or the pelvic wall.

In one embodiment, for clinical parameter "T grade", a T grade of 0 is given value=0 in the mathematical function, preferably in the multivariate Cox function, while a T grade of 1 to 4 is given value=1 in the mathematical function, preferably in the multivariate Cox function. Alternatively, specific values can be given for each grade above 0.

In one embodiment, at least one other clinical marker is used in the function. In particular, such marker is presence (or absence) of a gastro-intestinal disorder.

In particular, it is determined that the patient has gastro-intestinal toxicity (or gastro-intestinal disorder) if the patient has at least one of diarrhea, hemorrhoids, rectum inflammation (whether functional or physical) and when the trouble is graded 1 or more, according to the CTCAE v3.0 when relevant.

It is also possible to use, as the marker of gastro-intestinal toxicity, only one of the above marker, in particular rectum inflammation.

If the patient presents such gastro-intestinal toxicity, the value associated with this presence of the clinical marker in the function depends on the value of the grade as determined in the CTCAE v3.0.

If the patient presents no gastro-intestinal toxicity, the value associated with this presence of the clinical marker in the function is 0.

In one embodiment, another clinical marker is used in the function. In particular, such at least one other clinical marker is presence (or absence) of diabetes in the patient.

In one embodiment, another clinical marker is used in the function. In particular, such at least one other clinical marker is presence (or absence) of a gastro-intestinal disorder, preferably also taking into consideration the grade of such.

In one embodiment, another clinical marker is used in the function. In particular, such at least one other clinical marker is presence (or absence) of intake of anticoagulant by the patient, in particular linked to high blood pressure.

In one embodiment, the at least one other clinical parameter measured the method is neoadjuvant, concomitant and/or adjuvant hormonotherapy. As used herein, the term "neoadjuvant, concomitant and/or adjuvant hormonotherapy" refers to a treatment given after surgery, chemotherapy, and/or radiation therapy to lower the risk of recurrence of the cancer.

Examples of neoadjuvant, concomitant and/or adjuvant hormonotherapy include, but are not limited to, anti-androgens administration, administration of androgen synthesis inhibitors or administration of agonists of LHRH (luteinizing hormone-releasing hormone) or administration of GnRH antagonist. Examples of anti-androgens include, but are not limited to, flutamide, bicalutamide, nilutamide and enzalutamide. Examples of androgen synthesis inhibitors include, but are not limited to, ketoconazole, aminoglutethamide, abiraterone acetate and enzalutamide. Examples of LHRH agonists include, but are not limited to, leuprolide, goserelin, triptorelin and histrelin. Examples of GnRH antagonists include, but are not limited to, abarelix, degarelix, ozarelix and relugolix.

In one embodiment, the at least one clinical parameter measured in c) is the presence of at least one comorbidity before treatment, preferably selected from the group comprising high blood pressure, diabetes, angina pectoris, heart failure, chronic hepatitis, respiratory failure, arteritis, sexual disorders, and digestive disorders (disorders of the gastro-intestinal system).

In one embodiment, the at least one clinical parameter measured in c) is selected from the group comprising neoadjuvant, concomitant and/or adjuvant hormonotherapy, tobacco smoking and the presence of at least one comorbidity.

In one embodiment, for clinical parameter "tobacco smoking", a tobacco smoking patient is defined consistently as daily smoker, intermittent smoker or non-daily smoker (and given value=1 in the mathematical function, preferably in the multivariate Cox function), while a non-smoking patient is defined as some-day smoker or never daily smoker (and given value=0 in the mathematical function, preferably in the multivariate Cox function).Other classifications of patients could also be used.

In one embodiment, the clinical parameter is the presence or absence of lymph node dissection.

By "disease parameter" it is meant any parameter related to the disease (*e.g.*, prostate cancer) of the subject and relevant to assess an increased risk of radiation-induced toxicity in said subject. Examples of disease parameters include, but are not limited to, Gleason score, N grade (nodes according to the TNM classification), clinical characteristics of the prostate tumor (*e.g.*, size or volume of the tumor), and presence or absence of metastasis.

The Gleason grading system is based on microscopic appearance of prostate cancer. Gleason scores range from 2 to 10, with 2 representing the most-differentiated tumors and 10 the least-differentiated tumors. Tumors with Gleason scores of 8 to 10 are generally advanced neoplasms with low chances of cure.

In the TNM system, the "N" stands for lymph nodes. These bean-shaped organs help fight infection. Lymph nodes near the prostate in the pelvic region are called regional lymph nodes. Lymph nodes in other parts of the body are called distant lymph nodes. The different N grades are the following: N0: The cancer has not spread to the regional lymph nodes. N1: The cancer has spread to the regional (pelvic) lymph node(s).

In one embodiment, for clinical parameter "Gleason", a Gleason $\leq 7$ is given value=0 in the mathematical function, preferably in the multivariate Cox function, while a Gleason > 7 is given value=1 in the mathematical function, preferably in the multivariate Cox function.

In one embodiment, for clinical parameter "N grade", a N grade of 0 s given value=0 in the mathematical function, preferably in the multivariate Cox function, while a N grade of 1 or 2 is given value=1 in the mathematical function, preferably in the multivariate Cox function. Alternatively, specific values can be given for each grade above 0.

By "ionizing radiation treatment parameter" it is meant any parameter related to the ionizing radiation treatment planned to be applied to the subject and relevant to assess an increased risk of radiation-induced toxicity in the subject. Examples of ionizing radiation treatment parameters include, but are not limited to, ionizing radiation treatment energy range (*e.g.*, ranging from 6 to 25 MV), number of beam(s), technic used (either Intensity-modulated radiation therapy (IMRT) or 3D conformational radiation therapy (3D-RT)), boost (complement dose of irradiation), node irradiation, CTV, CTV1, CTV2, PTV, PTV1, PTV2, PTV1 maximal dose, PTV1 minimal dose, PTV1 mean dose, volume receiving 44 Gy, PTV2 maximal dose, PTV2 minimal dose, PTV2 mean dose, minimal dose received by 85% of the prescribed dose, volume PTV2 receiving 95% of the prescribed dose, volume of the rectal wall, volume of the bladder wall, volume of the intestinal contour, intestinal volume, maximal dose received by the rectal wall, dose received by 25% of the rectal volume, maximal dose received by the bladder wall, dose received by 25 or 50% of the bladder volume, dose received by 5% of the right or left femoral head, dose received by 70% of the penis bulb, mean dose received at the strip, treated volume (50 or 95% of the dose), volume of the anal canal, anal canal maximal dose, dose received by 25% or 50% of the volume of the anal canal.

In one embodiment, the method of the invention comprises measuring CTV, CTV1 and/or CTV2 in the subject.

The terms PTV and CTV are well known from the clinicians. The clinical target volume (CTV) comprises the position and extent of gross tumor, *i.e.*, what can be seen, palpated or imaged (which can be referred to as the gross tumor volume), plus a margin for sub-clinical disease spread which therefore cannot be fully imaged.

The planning target volume (PTV), allows for uncertainties in planning or treatment delivery. It is a geometric concept designed to ensure that the ionizing radiation dose is actually delivered to the CTV.

In one embodiment, the planning target volume (PTV) 1 may be defined as the prostate and seminal vesicles (proximal part in the absence of invasion) plus a 1 cm margin in all directions except posteriorly (5 mm), plus the CTV for lymph nodes and a 7 mm set-up margin when indicated. In case of a post-prostatectomy ionizing radiation, the planning target volume (PTV) 1 may be defined as the prostate bed plus a 1 cm margin in all directions except posteriorly (5 mm), plus the CTV for lymph nodes and a 7 mm set-up margin when indicated.

In one embodiment, the PTV2 consists of the prostate gland only or the prostate gland plus invaded seminal vesicles, with a 1 cm (5 mm posteriorly) margin. In case of a post-prostatectomy ionizing radiation, the PTV2 may consist of the prostate bed only with a 1 cm (5 mm posteriorly) margin.

In one embodiment, ionizing radiation treatment parameters as listed hereinabove are continued data, in particular parameters related to volumes, doses, etc...

In one embodiment, for the clinical parameters, disease parameters or ionizing radiation treatment parameters as listed hereinabove and that may be present or absent, the presence of said parameter is given value=1 in the mathematical function, preferably in the multivariate Cox function, while its absence is given value=0 in the mathematical function, preferably in the multivariate Cox function. For example, in one embodiment, at step (b), if the subject is treated by neoadjuvant, concomitant and/or adjuvant hormonotherapy, the value "1" is affected. In one embodiment, at step (b), if the subject is not treated by neoadjuvant, concomitant and/or adjuvant hormonotherapy, the value "0" is affected to the subject. In another embodiment, for continued data, the parameter is given its exact value in the mathematical function, preferably in the multivariate Cox function.

It is preferred to use a function that combines at least presence/absence of a urinary disorder and the tumor T grade.

It is preferred to use a function that combines only presence/absence of a urinary disorder and tumor T grade.

It is preferred to use a function that combines at least presence/absence of a urinary disorder, tumor T grade and presence/absence of a gastro-intestinal disorder.

It is preferred to use a function that combines at least presence/absence of a urinary disorder, tumor T grade, presence/absence of a gastro-intestinal disorder and tumor N grade.

It is preferred to use a function that combines at least presence/absence of a urinary disorder, tumor T grade and presence/absence of diabetes.

It is preferred to use a function that combines at least presence/absence of a urinary disorder, tumor T grade and presence/absence of intake of anticoagulants.

It is preferred to use a function that combines at least presence/absence of a urinary disorder, tumor T grade, presence/absence of diabetes, and presence/absence of a gastro-intestinal disorder.

It is preferred to use a function that combines at least presence/absence of a urinary disorder, tumor T grade, presence/absence of a gastro-intestinal disorder; tumor N grade and lymph node dissection.

It is preferred to use a function that combines at least presence/absence of a urinary disorder, tumor T grade, presence/absence of a gastro-intestinal disorder; tumor N grade and age of the patient.

It is preferred to use a function that combines at least presence/absence of a urinary disorder, tumor T grade, presence/absence of a gastro-intestinal disorder, tumor N grade, presence/absence of a concomitant hormonotherapy and level of PSA.

The present description also provides methods for obtaining a mathematical function that can be used in an in vitro non-invasive diagnosis test for assessing the risk of developing side effects after ionizing radiation in a subject suffering from prostate cancer.

These methods are performed on a cohort of patients that have received ionizing radiation treatment and for which occurrence of PSE have been reorded post-treatment.

These methods include:

a) classifying subjects of a cohort of patients into different groups according to the presence of side effects after ionizing radiation treatment

b) determining the presence or absence of urinary toxicity prior to ionizing radiation treatment,

c) determining the stage of the patient's tumor according to the TNM staging system

d) optionally determining the presence of at least one other marker selected from the group consisting in the presence or absence of concomitant and/or adjuvant hormonotherapy, the tobacco smoking habit, the presence or absence of gastrointestinal toxicity, the dosage of PSA (Prostate Specific Antigen), the presence of at least one comorbidity (in particular diabetes), the presence or absence of lymph node dissection, and the age of the patient, before ionizing radiation treatment

e) identifying by unidimensional analysis, the markers measured in step d) for which the measured value differs significantly between the group of subjects in which side effects after ionizing radiation have developed and the group of subjects in which side effects after ionizing radiation have not occurred, thereby obtaining independent factors

f) obtaining the mathematical function by combination of the factor measured in b), the marker determined in c) and the identified independent factors identified in e).

In order to obtain a function that is as accurate as possible, the number of patients in the cohort should be as large as possible, indicating that it preferably comprises more than 50 patients, preferably more than 100 patients, preferably more than 200 patients, more preferably more than 500 patients, or even more than 1000 patients. As indicated, there is no upper limit for the number of patients, and the larger, the better.

The duration of time is chosen by the person skilled in the art. If one desires that the function is indicative of the occurrence of side effect post treatment at two years, the duration of time shall be at least 2 years. This means that the groups of e) will be the patients with PSE after 2 years and the patients without PSE after 2 years. Other time periods can be used (1, 3, 5 years or more). In the present application, the disclosed functions have been designed with a 2 years period. Other functions can be designed, with other endpoint durations, which may be more accurate for these specific endpoints.

As indicated above the function may be a (multivariate) Cox function. Such Cox function is preferably obtained by

a) Assessing / evaluating the presence of PSE after a given duration of time in patients having had prostate cancer and having been treated by ionizing radiation treatment, wherein the values of the variables used in the function are known at the beginning of the duration of time (prior to treatment)
b) Performing a multivariate Cox regression by combination of said values, on the basis of the occurrence of PSE after the given duration of time
c) Assessing the independent discriminative value of the values by analysis of the multivariate Cox regression,
d) Combining the relative weight of the values of each marker, as individually determined in the multivariate Cox regression, with a negative sign when the markers harbor a negative correlation with the observation of PSE.

The markers that can be used in this method are the same as disclosed above. The period of time is chosen by the one of skill in the art, and is generally multiple years (such as 1, 2, 3 or 5 years).

It is to be noted that the different parameters that are proposed are either continuous (level of circulating PSA, age of the patient), or discrete with a binary classification (presence/absence of a comorbidity such as diabetes, or such as lymph node dissectoin) or a classification in multiple classes (such as the classification of the tumor grade according to the TNM classification, or of the grade of the urinary toxicity).

Consequently, the function obtained takes into consideration these differences between the inputed parameters. This implies that the values, in the function, for the parameters classified in discrete classes will depend on the class of the parameter.

As an illustration, for a function involving age of the patient (parameter a), a presence of urinary toxicity (parameter b), and tumor T stage (parameter c), a single coefficient $a1$ will be used and multiplied by the value found for the age (thereby representing a proportional contribution of the age in the function), various coefficients $b1$, $b2$, $b3$, $b4$ will be used depending on the stage of the urinary toxicity according to the CTCAE (grades 1 to 4 respectively) and various parameters $c1$, $c2$... will be used depending on the stage of the tumor (TNM staging).

In one embodiment, the *in vitro* method for assessing the risk of developing side effects after ionizing radiation in a subject, thus comprises the steps of:

a) determining the presence or absence, of urinary toxicity in the patient prior to application of ionizing radiation,
b) determining the stage of the patient's tumor,
c) optionally determining of at least one other clinical parameter, disease parameter or ionizing radiation treatment parameter from the patient, and
d) combining a value associated with the presence of urinary toxicity determined in a), a value associated with the stage of the tumor in b), and a value association with the at least one other clinical parameter, disease parameter or ionizing radiation treatment parameter determined in c) in a mathematical function to obtain an end-value.

In one embodiment, the *in vitro* method for assessing the risk of developing side effects after ionizing radiation in a subject, comprises the steps of:

a) determining the presence and optionally the grade of urinary disorders for the subject
b) determining the tumor T grade according to the TNM classification
c) determining tumor N stage, and optionally at least another parameter, and
d) combining values associated with the parameters measured or determined in a), b) and c) in a mathematical function in order to obtain an end value.

In a specific embodiment, the age of the subject is also included in the mathematical function of d).

In one embodiment, the *in vitro* method for assessing the risk of developing side effects after ionizing radiation in a subject, comprises the steps of:

a) determining the presence and optionally the grade of urinary disorders for the subject
b) determining the tumor T grade according to the TNM classification

c) determining the presence and optionally the grade of gastro-intestinal disorders for the subject, and optionally of at least another parameter

d) combining values associated with the parameters measured or determined in a), b), and c) in a mathematical function in order to obtain an end value.

In a specific embodiment, the age of the subject is also included in the mathematical function of d).

In one embodiment, the in vitro method for assessing the risk of developing side effects after ionizing radiation in a subject, comprises the steps of:

a) determining the presence and optionally the grade of urinary disorders for the subject

b) determining the tumor T grade according to the TNM classification

c) determining the presence or absence of a comorbidity, preferentially diabetes and/or intake of anticoagulants, and optionally at least another parameter, and

d) combining values associated with the parameters measured or determined in a), b), and c) in a mathematical function in order to obtain an end value.

In a specific embodiment, the age of the subject is also included in the mathematical function of d).

In one embodiment, the in vitro method for assessing the risk of developing side effects after ionizing radiation in a subject, comprises the steps of:

a) determining the presence and optionally the grade of urinary disorders for the subject

b) determining the tumor T grade according to the TNM classification

c) determining the presence or absence of a comorbidity, preferentially diabetes and/or intake of anticoagulants

d) determining the presence and optionally the grade of gastro-intestinal disorders for the subject

e) combining values associated with the parameters measured or determined in a), b), c), d) in a mathematical function in order to obtain an end value.

In a specific embodiment, the age of the subject is also included in the mathematical function of e).

In one embodiment, the mathematical function of the invention is a binary logistic regression, a multiple linear regression or any time-dependent regression.

In a preferred embodiment, the regression is a multivariate Cox regression.

In one embodiment, said regression is time-dependent, preferably is a time-dependent multivariate regression.

In one embodiment, said regression is a multivariate time-related model, preferably a Cox proportional hazard regression model. Such model is particularly well adapted for the purpose of the present invention, namely making a prognosis of the occurrence of an event (assessing the risk (or hazard) of the occurrence of such event) over time or after a given period of time, said event being the occurrence of side effect linked to the radiation treatment.

In one embodiment of the present invention, the independent parameters combined in the Cox regression are presence (optionally and preferably grades) of urinary disorders, and tumor T grade (according to TNM classification). Use of biochemical markers, clinical parameters, disease parameters and/or ionizing radiation treatment parameters related to the development of radiation-induced side effects, *e.g.*, PLE in a subject, neoadjuvant, concomitant and/or adjuvant hormonotherapy (0 if absent, 1 if present), tobacco smoking, comorbidities, and/or ionizing radiation treatment parameters (in particular CTV, CTV1 and/or CTV2), or other parameters as indicated above is also possible and the functions (and coefficient thereof) would be adjusted and modified depending on the other parameters included.

In one embodiment, the independent parameters combined in the Cox regression presence (or grade) of urinary disorders, tumor T stage, and optionally other biochemical markers, clinical parameters, disease parameters and/or ionizing radiation treatment parameters related to the development of radiation-induced side effects, as indicated above.

The multivariate Cox function may usually be obtained by combining the relative weight of each parameter, as individually determined in the multivariate Cox regression, with a negative sign when the markers harbor a negative correlation with the observation of PSE.

As indicated above, in order to define the multivariate Cox model of the invention (*i.e.*, "modelling"), a classification of cancer patients (preferably prostate cancer patients) is made based on the detection of radiation-induced side effects, preferably of PSE as described hereinabove, during the clinical follow-up of studies.

In one embodiment, said modelling may be based on a population (*e.g.*, a multicenter population) of prostate cancer patients treated by ionizing radiation (which may be named "reference population"). The steps to build up the model may thus consist in:

- the identification of biochemical markers and of clinical parameters, disease parameters or ionizing radiation treatment parameters relevant to assess an increased risk of radiation toxicity in a subject;

- the use of said identified biochemical markers and clinical parameters, disease parameters or ionizing radiation treatment parameters in a multicenter clinical trial to identify relevant variables as prognostic factors of radiation-induced side effects, in particular PSE, *i.e.*, as variables that are indicative of a specific risk to develop radiation-induced side effects, in particular PSE;
- the application of these variables on the large multicenter clinical trial to identify the predictive role of the combination of biochemical markers and of clinical parameters, disease parameters or ionizing radiation treatment parameters for developing radiation-induced side effects, in particular PSE.

By "multicenter research trial" it is meant a clinical trial conducted at more than one medical center or clinic.

In one embodiment, the multivariate Cox model is: Hazard (not experiencing a radiation-induced side effect at time t) = baseline hazard^exp(($\alpha$1*tumor T stage) + $\beta$1*(presence of urinary disorder) + $\beta$2*(Clinical parameter or disease parameter or ionizing radiation treatment parameter 2) + ... $\beta$n*(Clinical parameter or disease parameter or ionizing radiation treatment parameter (n) with n superior or equal to 2), where the baseline hazard corresponds to the hazard of not experiencing the event (the event being having PSE, not experiencing the event is remaining survival PSE-free) when all covariates are zero.

In another embodiment, the multivariate Cox model is:
Probability (experiencing a radiation-induced side effect before time t) = 1 - Probability (not experiencing a radiation-induced side effect before time t) = 1 - a^exp(($\alpha$1* tumor T stage) + $\beta$1*(presence of urinary disorder) + $\beta$2*(Clinical parameter or disease parameter or ionizing radiation treatment parameter 2) + ... $\beta$n*(Clinical parameter or disease parameter or ionizing radiation treatment parameter (n) with n superior or equal to 2) +$\gamma$).

As indicated above, in the context of the formulas of the present application, a given parameter can be classified in discrete classes X1 to Xm. In this case, the formula will include m coefficients $\beta$ for this class (for instance $\beta$1 to $\beta$m). The value associated with each $\beta$i coefficient will be "if said parameter is in class i, then value of "Clinical parameter or disease parameter or ionizing radiation treatment parameter" is equal to 1, whereas it is equal to 0 if said parameter isn't in class i.

As an illustration, when there are 4 classes for a parameter, if the parameter for the patient is in class 3, only the $\beta$3 coefficient will be taken into consideration, the other ones $\beta$1, $\beta$2 and $\beta$4 being nullified. This is in particular valid for the urinary toxicity included in the formula, as the symptoms can be classified in four classes according to the CTCAE. It is also valid for the tumor T stage.

The right-hand side of the above equation specify the underlying function of the model. The left-hand side of the equation is the predicted probability. It may be presented in a nomogram and communicated to the patient, in particular to provide an informed consent to the proposed and selected therapy. Alpha and beta coefficients must be estimated for each covariate and converted to hazard ratios as a measure of effect, as in any statistical report. To obtain the predicted probability of the event in question (experiencing a radiation-induced side effect,), the above equation is calculated using a patient's individual characteristics and the model-derived beta coefficients.

In one embodiment, the baseline hazard at time t corresponds to the basal risk to develop a radiation-induced side effect, with all co-variables considered as reference value (equal to 0).

Clinical parameters '1' to 'n' may be selected in the list of clinical parameters or disease parameters or ionizing radiation treatment parameters as disclosed hereinabove. In one embodiment, said clinical parameters or disease parameters or ionizing radiation treatment parameters include neoadjuvant, concomitant and/or adjuvant hormonotherapy, tobacco smoking, at least one comorbidity, and/or at least one ionizing radiation treatment parameter (in particular CTV, CTV1 and/or CTV2). In one embodiment, said clinical parameters or disease parameters or ionizing radiation treatment parameters include neoadjuvant, concomitant and/or adjuvant hormonotherapy, at least one comorbidity, and/or at least one ionizing radiation treatment parameter (in particular CTV, CTV1 and/or CTV2). In one embodiment, said clinical parameters or disease parameters or ionizing radiation treatment parameters include neoadjuvant, concomitant and/or adjuvant hormonotherapy.

In one embodiment, Hazard (experiencing a radiation-induced side effect) = 1-a^exp(($\alpha$1* biochemical marker) + $\beta$1*(Clinical parameter or disease parameter or ionizing radiation treatment parameter 1) + $\beta$2*(Clinical parameter or disease parameter or TIR parameter 2) + ... $\beta$n*(Clinical parameter or disease parameter or ionizing radiation treatment parameter (n) with n superior or equal to 2) + $\gamma$). The baseline hazard corresponds to the hazard of experiencing the event (PSE) when all covariates are zero. In one embodiment, the value entered for a given parameter in the formula hereinabove is 0 if said parameter is absent and 1 if said parameter is present (such as, for example, for the presence or absence of a comorbidity). In another embodiment, the value entered for a given parameter in the formula hereinabove corresponds to the measured value of said parameter. This embodiment applies to parameters measured as continued data, such as, for example, CTV, CTV1 and CTV2.

In one embodiment, Hazard (experiencing a radiation-induced side effect) =1 - a^exp($\beta$1*(patient with class 1 urinary toxicity [0=no; 1=yes]) + $\beta$2*(patient with class 2 urinary toxicity [0=no; 1=yes]) + $\beta$3*(patient with tumor T1B class [0=no; 1=yes]) + $\beta$4*(patient with tumor T1C class [0=no; 1=yes]) + $\beta$5*(patient with tumor T2A class [0=no; 1=yes]) + β6*(patient with tumor T2B class [0=no; 1=yes]) + β7*(patient with tumor T2C class [0=no; 1=yes]) + β8*(patient with tumor T3 class [0=no; 1=yes]) + β9*(patient with tumor T3A class [0=no; 1=yes]) + γ).

The γ constant thus corresponds to the pondered sum of the average values in the population and the formula thus allows to detect the variation, for a patient, with regards to the mean of the data in the population. Thus, the risk or probability detected by the function is relative with regards to the population.

In one embodiment, Hazard (experiencing a radiation-induced side effect) may also be named risk to develop a radiation-induced side effect, for a subject.

In one embodiment, based on this multivariate Cox function, the skilled person would be able to introduce any additional relevant biochemical marker(s) and/or clinical, disease or ionizing radiation treatment parameter(s) to said multivariate Cox model.

In one embodiment, the different coefficients used for the values obtained for the different markers in the function of the invention, preferably in the multivariate Cox regression can be calculated through statistical analysis in a reference population of patients.

In one embodiment, the method of the invention thus comprises measuring an end-value, wherein said end-value is indicative of the risk of the subject to develop a radiation-induced side effects described hereinabove. In one embodiment, said risk is estimated taken into account all co-variables (biochemical markers and clinical parameters, disease parameters and/or ionizing radiation treatment parameters, which are combined in a mathematical function).

Therefore, in one embodiment, the "end-value" is the linear-predictor allowing the calculation of probability of experiencing a radiation-induced side effect for each subject.

In one embodiment, depending on the end-value obtained for a subject, it is possible to predict for said subject the risk of developing a radiation-induced side effect, during follow-up after ionizing radiation treatment, such as, for example, 3 months or 6, 12, 18, 24, 30 or 36 months after the end of the ionizing radiation treatment. The higher the end-value, the higher the risk of developing PSE.

In another embodiment, the choice of the optimal model of the invention is assessed by the Harrell's C-index for censored observations and is equal to the probability of concordance between two survival distributions (Harrell and Shih 2001). The C-index or concordance index quantifies the level of concordance between predicted probabilities and the actual chance of having the event of interest. In one embodiment, the method of the invention has a Harrell's C-index of at least about 0.5, preferably of at least about 0.55, more preferably of at least about 0.56, 0.57, 0.58 or more. In one embodiment, the method of the invention has a Harrell's C-index of at least about 0.585 or 0.590.

In the preferred embodiment, the choice of the optimal model of the invention is assessed by the Area Under the Receiving Operating Curve (AUROC). It is preferred when the formula has an AUROC higher than 0.63, more preferably equal or higher than 0.64, more preferably equal or higher than 0.65, more preferably equal or higher than 0.66, more preferably equal or higher than 0.67, more preferably equal or higher than 0.68, more preferably equal or higher than 0.69, most preferably equal or higher than 0.7.

In one embodiment, depending on the end-value obtained for a subject, it is possible to predict for said subject the risk of developing a radiation-induced side effect, during follow-up after ionizing radiation treatment, such as, for example, 3 months or 6, 12, 18, 24, 30 or 36 months (in particular 24 months) after the end of the ionizing radiation treatment. This is is a relative risk, when compared to the population of patients. The higher the end-value, the higher the risk of developing PSE.

By making a threshold vary from 0 to 1, it is possible to plot the AUROC (calculating the sensitivity and the specificity for each threshold). The choice of a threshold makes it possible to obtain a reference end-value that can be used by the physician to decide whether the patient has a "clinical" risk of developing PSE (if the end-value for the patient is higher than the reference end-value) or not (if if the end-value for the patient is below the reference end-value). One can note that different reference end-values can be be selected, depending on whether one want to maximize the sensitivity (Se, detection of true positives), specificity (Sp, nondetection of true negatives), positive predictive value (PPV, probability that the patient is not a false positive), or negative predictive value (NPV, probability that the patient is not a false negative). Generally, it is interesting, for such methods, to maximize the NPV, in order to lower the risk of PSE for patients offered with a radiation treatment. Alternatively, selecting a reference end-value to maximize the Se makes it possible to propose alternative treatment to patients with a risk of making PSE, but this would generally be balanced by a lower Sp. The method is very interesting to stratify (or classify) patients, so as to be able to propose a treatment that is adapted and specific to the patient. Thus, and as described below, depending on the risk of the patient, and generally on the risk of tumor recurrence, the physician will be able to propose a treatment that would maximaze the chances of cure, and minimize the secondary effects. Thus, the methods herein disclosed are of great interest in providing a help to the physician when investigating a clinical case. Consequently, even when obtaining an end-result and comparing it with a reference threshold, the physician shall also take into account the clinical and general context to be able to propose the treatment.

In one embodiment, the method of the invention comprises comparing the end-value obtained for a subject with a reference end-value.

As seen above, it is preferred when the end-value is compared to the reference value estimated by the model which discriminate patients with PSE and patients without PSE.

If the end-value is higher to the reference end-value, patients is at high level of risk to develop a PSE, in particular when the end-value is more than 10% higher than the reference end-value.

If the end-value is lower than the reference end-value, patients is at low level of risk to develop a PSE, especially when the end-value is more than 10% lower than the reference end-value.

In one embodiment, the method of the invention is computerized (or computer-implemented).

Another object of the present invention is thus a computer software for implementing the method of the invention.

In one embodiment, the method of the invention is implemented with a microprocessor comprising a software configured to calculate an end-value resulting from the combination of the measures of the presence (and preferably the grade) of urinary disorders pre-treatment, the tumor T stage, and optionally of at least one clinical parameter, disease parameter and/or ionizing radiation treatment parameter as disclosed above, (preferably neoadjuvant, concomitant and/or adjuvant hormonotherapy, tobacco smoking, at least one comorbidity and/or at least one ionizing radiation treatment parameter (in particular CTV, CTV1 and/or CTV2), more preferably neoadjuvant, concomitant and/or adjuvant hormonotherapy).

Another object of the present invention is directed to a system including a machine-readable memory, such as a computer and/or a calculator, and a processor configured to compute said mathematical function, in particular said multivariate Cox function, and provide the end-value for a given patient. This system may be dedicated to perform the method according to the invention.

In one embodiment, the method of the invention thus comprises:

- determining the presence or absence of urinary disorder (toxicity) and preferably the CTCAE 3.0, and the T stage of the tumor (TNM classification)
- measuring at least one other biochemical marker and/or at least one other clinical parameter, disease parameter or ionizing radiation treatment parameter, such as the ones disclosed herein, preferably neoadjuvant, concomitant and/or adjuvant hormonotherapy, gastro-intestinal disorders, lymph node dissection, tumor N stage, tobacco smoking, at least one comorbidity and/or at least one ionizing radiation treatment parameter (in particular CTV, CTV1 and/or CTV2), more preferably neoadjuvant, concomitant and/or adjuvant hormonotherapy, for all subjects of a reference population;

- univariate analysis (estimation for each parameter one by one in order to select all significant parameters with p-value ≤ 0.2) under a Cox regression model;
- multivariate analysis (estimation including all selected parameters by univariate analysis + adding optional non-significant parameters which are clinically relevant) under Cox regression model;
- selection of significant parameters and/or clinically relevant to obtain the final model whose linear predictor were extracted to estimate the risk (probability) to develop a radiation-induced side effect; linear predictor were integrated in a software;

According to one embodiment, the method of the invention comprises implementing the data obtained in step a) and in step b) to a computer or a calculator that will calculate the multivariate Cox regression and the risk of developing of a radiation-induced side effect. The data obtained by the physician is therefore more easily interpretable, and will allow for an improvement in the process for deciding the adapted patient care.

The invention also relates to a device for the prognosis of the risk of a patient to have PSE, comprising a first means, wherein the first means provides an end value by combining the parameters as indicated above through a mathematical function as disclosed herein.

The functions and markers to be implemented within this device are the ones disclosed in the present specification. As herein disclosed the device preferably makes it possible to obtain the end value using the functions disclosed above.

Another object of the present invention is a kit for collecting data of a subject to be further used for detecting the risk of developing of a radiation-induced side effect in said subject using the method of the present invention, wherein the kit comprises:

- a box/container and bag suited for biological transportation of a patient's sample, in particular a blood sample; and
- forms to be completed by the patient and/or the nurse and/or the physician, specifically designed and necessary to implement the method of the invention and provide the necessary information to be combined in the formula.

As an example, the forms may contain specific questions aimed at collecting information necessary to run the predictive analysis such as, age, whether the patient has undergone or will undergo an adjuvant treatment (chemotherapy or concomitant hormone therapy, for example), tobacco smoking habit, comorbidities, whether the patient has urinary toxicity, whether the patient has gastro-intestinal toxicity, whether the patient has diabetes, whether the patient takes anticoagulants, whether the patient has had lymph node dissection, characteristics of the ionizing radiation treatment planned to be applied to the subject and date and time when the sample was recovered.

Another object of the present invention is a kit for detecting the risk of developing a radiation-induced side effect, in a subject using the method of the present invention, wherein said kit comprises:

- means for collecting information on the presence/absence or urinary disorders and of the tumor T stage;
- optionally means for collecting information on at least one clinical parameter, disease parameter or ionizing radiation treatment parameter according to the invention, such as a survey.

By "kit" is intended any manufacture (*e.g.*, a package or a container). The kit may be promoted, distributed, or sold as a unit for performing the methods of the present invention. Furthermore, any or all of the kit reagents may be provided within containers that protect them from the external environment, such as in sealed containers. The kits may also contain an instructional material describing the kit and methods for its use. Kits are also provided that are useful for various purposes. The label or instructional material may provide a description of the content of the kit as well as instructions for the intended use.

In one embodiment, the means for collecting information on the presence/absence or urinary disorders and of the tumor T stage and on at least one clinical parameter, disease parameter or ionizing radiation treatment parameter according to the invention correspond to specific forms to be completed by the patient and/or the nurse and/or the physician, specifically designed and required to run the method of the invention and the nomogram analysis. In a preferred embodiment, these forms may contain specific questions aimed at collecting information necessary to run the predictive analysis such as age, whether the patient has undergone or will undergo an adjuvant treatment (hormonotherapy, for example), tobacco smoking habit, comorbidities, whether the patient has urinary toxicity, whether the patient has gastro-intestinal toxicity, whether the patient has diabetes, whether the patient takes anticoagulants, whether the patient has had lymph node dissection, parameters of the ionizing radiation treatment planned in the subject and date and time when the T cell containing sample was recovered.

In one embodiment, the kit for detecting the risk of developing radiation-induced side effects (*e.g.*, PSE as described hereinabove) in a subject comprises:

- specific forms to be completed by the patient and/or the nurse and/or the physician to record the presence/absence or urinary disorders and the tumor T stage; and
- optionally specific forms to be completed by the patient and/or the nurse and/or the physician, specifically designed and required to run the method of the present invention.

In one embodiment, determining the end-value for a subject will help the physician to adapt the dose and sequences of ionizing radiation treatment to the subject to limit the radiation-induced side effects, and optionally to adapt the treatment by replacing ionizing radiation treatment with other therapeutic treatments.

In one embodiment, the end-value (for example obtained with a multivariate Cox function) is used for the choice of a suitable treatment for the patient, such as an appropriate ionizing radiation treatment regimen, or surgery.

In one embodiment, the end-value is used for the choice of a suitable treatment for the patient or a suitable patient care, such as an appropriate ionizing radiation treatment dosage regimen, wherein:

- if the patient presents a risk to develop radiation-induced side-effects,the appropriate ionizing radiation treatment dosage regimen may be decreased for example by delivery of prostate hypofractionated treatment. Alternatively, in some embodiments, ionizing radiation treatment may be avoided completely (or postponed) for individuals with a high risk of toxicity, provided that an effective alternative exists. More specifically, for prostate cancer, surgery could be offered instead of ionizing radiation treatment for patients with high risk of toxicity. Patients with high-risk of toxicity could also be offered protontherapy treatment instead of conventional photontherapy treatment, or pelvic therapy, or MRI linac with reduced margin, or ionizing radiation treatment with rectal spacer or transponders (*e.g.*, with reduced margin), focal therapy or brachytherapy;
- if the patient presents low risk or no risk to develop radiation-induced side-effect, the patient might be offered a usual ionizing radiation treatment dosage regimen or a higher dose with modern techniques or ionizing radiation treatment in combination with systemic therapies with the aim of improving local control (*e.g.*, treatment with chemotherapy or radiosensitizers), or hypofractionated schedules (allowing decreased treatment duration) or dose escalation of ionizing radiation treatment (optionally with boost brachytherapy) or pelvic radiation therapy or stereotactic body radiation therapy.

As seen above, if the end-value is higher to the reference end-value, patients is at high level of risk to develop a PSE, in particular when the end-value is more than 10% higher than the reference end-value.

If the end-value is lower than the reference end-value, patients is at low level of risk to develop a PSE, especially when the end-value is more than 10% lower than the reference end-value.

Another object of the invention is a method for implementing an adapted patient care for a patient (preferably a prostate cancer patient), wherein said method comprises:

- assessing the risk for said patient to develop radiation-induced side-effects, using the *in vitro* method as described hereinabove;
- implementing an adapted patient care depending on the risk for the patient to develop radiation-induced side-effects.

In one embodiment, the patient presents a risk (*e.g.*, a high risk) to develop radiation-induced side-effect, and the adapted patient care is selected from a decreased ionizing radiation treatment dosage regimen (*e.g.*, delivery of prostate hypofractionated treatment), absence of ionizing radiation treatment (or postponement of ionizing radiation treatment), surgery, protontherapy treatment instead of conventional photontherapy treatment, MRI linac with reduced margin, ionizing radiation treatment with rectal spacer or transponders (*e.g.*, with reduced margin), pelvic therapy, focal therapy or brachytherapy.

In one embodiment, the patient presents a low risk or no risk to develop radiation-induced side-effect, and the adapted patient care is selected from a usual ionizing radiation treatment dosage regimen, or a higher dose of ionizing radiation treatment with modern techniques, dose escalation of ionizing radiation treatment (optionally with boost brachytherapy), pelvic ionizing radiation treatment, ionizing radiation treatment in combination with systemic therapies with the aim of improving local control (*e.g.*, treatment with chemotherapy or radiosensitizers), hypofractionated schedules (allowing decreased treatment duration) or stereotactic body ionizing radiation treatment.

In one embodiment, the patient presents a high risk of tumor recurrence and a low risk to develop radiation-induced side-effect, and the adapted patient care is selected from hypofractionation of ionizing radiation treatment and stereotactic body ionizing radiation treatment.

In one embodiment, the patient presents a low risk of tumor recurrence and a low risk to develop radiation-induced side-effect, and the adapted patient care is selected from dose escalation of ionizing radiation treatment (optionally with boost brachytherapy) and pelvic ionizing radiation treatment.

In one embodiment, the patient presents a high risk of tumor recurrence and a high risk to develop radiation-induced side-effect, and the adapted patient care is selected from focal therapy, brachytherapy, ionizing radiation treatment with rectal spacer or with transponders with reduced margin.

In one embodiment, the patient presents a low risk of tumor recurrence and a high risk to develop radiation-induced side-effect, and the adapted patient care is selected from surgery, ionizing radiation treatment with rectal spacer or with transponders, pelvic therapy or proton therapy.

Methods to assess the risk of tumor recurrence are well-known by the skilled artisan and include, without limitation, the use of the classification developed by D'Amico et al (JAMA, 1998, 280(11):969-74). The methods for determining / proposing a treatment are also described in Azria et al (Front Oncol. 2017 Apr 27;7:83).

## EXAMPLES

The present invention is further illustrated by the following examples, which are part of the invention.

Example 1:

***Materials and methods***

*Patient analysis*

Analysis was performed among 383 prostate cancer patients treated by radiotherapy (*i.e.*, ionizing radiation treatment). Patients were followed with a median follow-up of 38.2 months. A specific questionnaire (Case Report Form) was filled out for each patient including general social demographics administrative data, risk factors, and at each visit, clinical and biological and treatment items, as described in **Table 1**. 240 patients were followed for at least 36 months according to the protocol.

*Preparation and Delivery of Radiotherapy*

Patients underwent CT-based virtual simulation (CT Simulator, General Electric, Cleveland, OH) with 2.5 mm thick slices obtained at 2.5 mm intervals in the supine position. A small flexible rectal tube was inserted to evacuate flatus, and then removed. Intravenous contrast was used in all patients to permit better delineation of pelvic lymph nodes and bladder. Patients were positioned with knee and feet support (Sinmed, The Netherlands) but no custom immobilization device was used. The isocenter was set in the middle of the prostate using our virtual simulation console (AdvantageSim, General Electric, Cleveland, OH) by the treating physician immediately after the first scan, while the patient waited in the treatment position on the scan table. The isocenter was then tattooed on the patient skin using our scan room mobile lasers (LAP Dorado CT-4).

Structures were manually contoured on the CT scan. When pelvic irradiation was indicated, the clinical target volume (CTV) for the lymph nodes included the obturator, internal iliac, external (excluding the artery) iliac and sacral vessels plus a circumferential 7 mm margin in accordance with the RTOG consensus guidelines for contouring.

The planning target volumes (PTV) 1 was defined as the prostate and seminal vesicles (proximal part in the absence of invasion) plus a 1 cm margin in all directions except posteriorly (5 mm), plus the CTV for lymph nodes and a 7 mm set-up margin when indicated.

In case of a post-prostatectomy radiotherapy, the planning target volumes (PTV) 1 was defined as the prostate bed plus a 1 cm margin in all directions except posteriorly (5 mm), plus the CTV for lymph nodes and a 7 mm set-up margin when indicated.

The PTV2 consisted of the prostate gland only or the prostate gland plus invaded seminal vesicles, with a 1 cm (5 mm posteriorly) margin.

In case of a post-prostatectomy radiotherapy, the PTV2 consisted of the prostate bed only with a 1 cm (5 mm posteriorly) margin.

The bladder was contoured in its entirety. The rectum was contoured as a whole organ but starting 2 cm above and below the CTV. Femoral heads were drawn from the top of the acetabulum to the small trochanter inferiorly. Small bowel was determined in all slices where the PTV was apparent. To take interfractional bowel motion into account, the small bowel was delineated as a whole pelvic and abdominal cavity excluding bones, muscle and other organs at risk (OAR), rather than contoured as individual bowel loops. For bowel, rectum and bladder, a second volume was created and defined as the considered organ minus the PTV (bowel - PTV, rectum - PTV, bladder - PTV) to avoid hot spots and improve optimization.

In case of prostate in place, the prescribed doses to the PTV1 and PTV2 were 54 Gy and 74-80 Gy in 37-40 daily fractions, respectively.

In case of radiotherapy post-prostatectomy, the prescribed doses to the PTV1 and PTV2 were 54 Gy and 66-70 Gy in 37 daily fractions, respectively.

RapidArc® plans were performed using the Eclipse software version 8.9.08 (Helios, Varian, Palo Alto, CA). A maximum dose rate of 600 MU/min and 18 MV photon beams were selected. RapidArc with 1 arc (RA1) corresponded to a single 360° rotation, and RapidArc with 2 arcs (RA2) to two coplanar arcs of 360° sharing the same isocenter and optimized independently and simultaneously. These two arcs were delivered with opposite rotation (clock and counter-clock) so that off-treatment between the two beams was minimized to about 25 seconds. For RA1, field size and collimator rotation were determined by the automatic tool from Eclipse to encompass the PTV. We controlled that the collimator was rotated to a value different from zero in order to avoid the tongue-and-groove effect. For RA2, the first arc was similar to that defined in the RA1 process except for the rotation of the collimator, which was 360-X for the second arc (X corresponded to the rotation of the collimator of the first arc).

Optimization process was undertaken by decreasing as much as possible the dose to OAR without altering PTV coverage, and the results were improved by modifying constraints and priority factors. These parameters were modified with regard to the DVH results for each patient. We always checked that a good PTV2 coverage by the 74-80 Gy isodose was obtained, 95% of the PTV receiving at least 95% of the prescribed dose. Regarding rectal dose, the maximal dose allowed was 74-80 Gy, the volume receiving 72 Gy (V72) should remain less than 25 % and the V60 less than 50 %. For bladder, the maximal dose was 74 Gy, V70 should remain less than 50 % and V75 less than 25 %. The maximal dose to the femoral heads was 55 Gy and V50 was less than 5 %. The maximal dose allowed for small bowel was 34 Gy, with less than 450 cc receiving 30 Gy or more and less than 200 cc receiving 40 Gy or more.

VMAT plans with one arc or 2 arcs were optimized to try to achieve the best results with the possibility offered by the software.

*Adjuvant Systemic Therapies*

The LHRH agonist or GnRH antagonist, was administered subcutaneously every 4 weeks or every 3 months or every 6 months starting before or during radiotherapy and continued for 4 months to 3 years; In some patients, a steroidal antiandrogen was given orally for 1 month starting a week before LHRH agonist to prevent the flare that results from the testosterone surge occurring after LHRH agonist administration.

*End-point Assessments: identification of biomarkers and relevant covariables as prognostic factors of PLE on the reference population*

Toxicity evaluations were performed at baseline, every week during RT, one, three and six months after the last RT fraction, every 6 months up to month 36. The severe grade $\geq$2 PLE observed during the follow-up after RT was considered as the primary endpoint. The other severe late effects (lung, cardiac, skin, lung, for example) observed from 12 weeks to 3 years post RT and the most severe acute side effects observed from the start of RT to 12 weeks post RT were considered as the secondary endpoints. Toxicities were evaluated using all the possible definitions described by (Trotti, Colevas et al. 2003).

All endpoints were defined as the interval between the start of RT and following the first events: death for Overall Survival (OS), local or distant recurrence or death for Relapse-Free Survival (RFS), grade $\geq$2 PLE for Complication-Free Survival (CFS), and first event of RFS and C-FS for Complication-Relapse Free Survival (C-RFS) (Peto, Pike et al. 1977). Censoring patients were patients alive at the last follow-up visit for OS, patients alive and without relapse for RFS, patients alive who never experienced a grade $\geq$2 PLE for C-FS and patients alive who never experienced grade $\geq$2 PLE or relapse for C-RFS.

*Sample Size Calculation and Statistical Analysis*

Details are presented in the following protocol. Briefly, based on o2 =0.54, an estimated gastrointestinal complication rate of $\psi$ =5% and genitourinary complication rate of $\psi$=10%, with a two-sided $\alpha$ error of 0.05 and a $\beta$ error of 0.05 (power=0.95), 368 patients had to be included.

o2 is the variance of the studied variable (logCD8) and $\psi$ is the rate of complication/toxicity expected events.

The cumulative incidences of complications as a function of the prognostic variables were calculated using a non-parametric model (Pepe and Mori 1993). For multivariate analysis, selected factors were the baseline parameters with a p-value (statistical significance) less than 0.20 in univariate analysis. Final model was defined using backward stepwise selection (p<0.15) and a step by step method was used to include only the significant parameters (p<0.05) or clinically relevant and/or (p<0.10).

Data were summarized by frequency and percentage for categorical variables and by median and range for continuous variables.

OS, RFS, C-FS and C-RFS rates were estimated by the Kaplan-Meier method (Kaplan and Meier 1958). Ninety-five percent confidence intervals (95%CI) were also determined.

Univariate analysis and multivariate analysis were performed using the Cox proportional hazard's regression model (Cox, et al. 1984) to estimate the hazard ratio including baseline characteristics and treatment parameters. Comparisons were performed using the log-rank test for univariate analysis. Independent effects were evaluated from the likelihood ratio statistics.

Impact of the parameters on complication -relapse-free survival (C-RFS) was assessed. The cumulative incidence of PLE and relapse or death were estimated from a competing risk model using estimates obtained from the cause-specific hazard functions and the composite RFS and C-FS distribution (Arriagada, Rutqvist et al. 1992) and compared using Gray's test.

Median follow-up was estimated with the inverse Kaplan-Meier method. A p-value less than 0.05 was considered as significant. All statistical tests were two-sided. Stata was used for all statistical analyses (version 13.0) and the SAS macro %cif was used for Gray's test.

To complement analysis, receiver-operator characteristic (ROC) curve analyses for various parameters combination were performed to identify patients who experienced at least a grade 2 PLE during the follow-up (Kramar, Faraggi et al. 2001). The empirical areas under the ROC curves (AUC) and the respective 95%CI were used to determine the sensitivity, specificity, positive predictive value (PPV), and negative predictive value (NPV).

### Results

*Descriptive statistical analysis for modelling*

The analysis was performed on selected data from studies for all patients observing independent variables, as presented in **Table 1** below.

Table 1

|  | N=352 ('reference population') |
|---|---|
| **Age (Year)** | |
| Median (range) | 69.00 (49.00:88.00) |
| **Weight (kg)** | **n = 339** |
| Median (range) | 79.0 (58.0 :160.0) |
| **PSA dosing** | **n=335** |
| Median (range) | 8.4 (0.00:86.3) |
| **Gleason** | **n=342** |
| 5-6<br>7<br>8-9-10 | 116<br>176<br>50 |
| **High blood pressure** | |
| No<br>Yes | 201<br>151 |
| **Diabetes** | |
| No<br>Yes | 301<br>51 |
| **Angina pectoris** | |
| No<br>Yes | 342<br>10 |
| **Heart failure** | |
| No<br>Yes | 328<br>24 |
| **Chronic hepatitis** | |
| No<br>Yes | 345<br>7 |
| **Respiratory failure** | |
| No<br>Yes | 343<br>9 |
| **Arteritis** | |
| No<br>Yes | 345<br>7 |
| **Hypercholesterolemia** | |
| No<br>Yes | 282<br>70 |
| **Tobacco smoking** | **n=337** |
| Non smoker<br>Ex-smoker or Smoker | 140<br>197 |
| **Adjuvant Hormonotherapy** | |
| No<br>Yes | 162<br>190 |

(continued)

| Adjuvant Hormonotherapy | |
|---|---|
| Agonists LHRH<br>No<br>Yes | 5<br>185 |
| Antiandrogens<br>No<br>Yes | 132<br>56 |
| **Anticoagulants** | |
| No<br>Yes | 256<br>96 |
| **Sexual disorders** | |
| Grade 0-1<br>Grade 2-3-4 | 299<br>25 |
| **Urinary disorders** | |
| Grade 0-1<br>Grade 2-3-4 | 340<br>11 |
| **Digestive disorders** | |
| Grade 0-1<br>Grade 2-3-4 | 347<br>4 |
| **At least one disorder (among sexual, urinary and digestive disorders)** | |
| Grade 0-1<br>Grade 2-3-4 | 288<br>63 |

According to the CTCAE V3.0 (Trotti, Colevas et al. 2003), toxicity was graded according to severity of symptoms.

The main endpoint was the identification of probability to develop a PLE during 3 years follow-up.

The first stage consisted in identification of prognostic factors of development of PLE using univariate analysis and using the log rank test.

The second stage consisted in analysis of multivariate Cox proportional hazard model to assess the independent parameters for the prediction of the occurrence of PLE and to estimate the effect size defined as Hazard ratio (HR).

The best model was selected using the highest AUROC.

For such prognosis of a responding phenotype, a responding condition or test outcome is considered a positive result, while a non-responding condition or test outcome is considered a negative result. True and false positive results, NPV, PPV, specificity, sensitivity are defined and calculated as follows (Table 2):

**Table 2**

| | | Condition (responding) | |
|---|---|---|---|
| | | *Positive* | *Negative* |
| **Test outcome (responding)** | *Positive* | True Positive (TP) | False positive (FP) |
| | *Negative* | False negative (FN) | True negative (TN) |
| PPV = TP / (TP+FP)<br><br>NPV = TN / (TN+FN)<br><br>Sp = TN / (TN+FP)<br><br>Se = TP / (TP+FN) | | | |

"ROC" or "ROC curve" is a tool for diagnostic/prognostic test evaluation, wherein the true positive rate (Sensitivity) is plotted in function of the false positive rate (1-Specificity) for different cut-off points of a parameter after classification of patients. Each point on the ROC curve represents a sensitivity/specificity pair corresponding to a particular decision threshold (from 0 to 1). The area under the ROC curve (AUC) is a measure of how well a parameter can distinguish between two diagnostic/prognostic groups (diseased/normal). The accuracy of the test depends on how well the test separates the group being tested into those with and without the disease in question. Accuracy is measured by the area under the ROC curve. An area of 1 represents a perfect test; an area of 0.5 represents a worthless test.

It is usually acknowledged that a ROC area under the curve (AUROC) has a value superior to 0.7 is a good predictive curve for diagnosis/prognosis, although an AUROC higher than 0.63 also indicates a function with a relatively good quality. The ROC curve has to be acknowledged as a curve allowing prediction of the diagnosis quality of the method.

## Example 2. Generation of predictive functions

Multiple Cox functions were generated, combining the tumor T stage with presence of urinary disorders (patients classified according to the CTCAE grade of the toxicity) alone or in combination with other parameters. The data showed that combining tumor T stage with presence of urinary disorders is found in all combinations that are of good prognosis quality. Adding other parameters (such as presence of tumor N stage, lymph node dissection, diabetes, age of the patient, presence of gastro-intestinal disorders, intake of anticoagulants...) also made it possible to obtain functions with good prognosis value, as attested by AUROC higher than 0.63, or even higher than 0.70.

This example illustrates various embodiments of the invention. all functions herein disclosed are also subject of the invention. These functions are quantitative, i.e., the higher the end-result, the higher the relative risk of having PSE within 24 months.

A Cox function was developed using the T stage of tumor (TNM staging system) and the presence/absence and grade (CTCAE v3.0) of urinary toxicities. The AUROC for this function was 0.640. With a cutoff determined to maximize sensitivity and specitivity, the function showed the sensitivity of 0.493, specificity is 0.765, positive predictive value is 0.508 and negative predictive value is 0.754. In view of the NPV, the function can thus be used to identify patients with low risk of having PSE and to which a radiation treatment can be proposed.

Another model was developed by using the presence/absence of diabetes (comorbidity). This made it possible to obtain a function F1:

$$F1 = 1 - (1-a)^{\exp(f1)}$$

with f1 = β1*tumt1B + β2*tumt1C + β3*tumt2A + β4*tumt2B + β5*tumt2C + β6*tumt3 + β7*tumt3A + β8*tumt3B + β9*URI1 + β10*URI2 + β11*DIAB + γ

In this function f1

tumt1B has value 1 if the tumor T grade of the patient is 1B and 0 if not
tumt1C has value 1 if the tumor T grade of the patient is 1C and 0 if not
tumt2A has value 1 if the tumor T grade of the patient is 2A and 0 if not
tumt2B has value 1 if the tumor T grade of the patient is 2B and 0 if not
tumt2C has value 1 if the tumor T grade of the patient is 2C and 0 if not
tumt3 has value 1 if the tumor T grade of the patient is 3 and 0 if not
tumt3A has value 1 if the tumor T grade of the patient is 3A and 0 if not
tumt3B has value 1 if the tumor T grade of the patient is 3B and 0 if not
URI1 has value 1 if the urinary toxicity is of grade 1 and 0 if not
URI2 has value 1 if the urinary toxicity is of grade 2 and 0 if not.
DIAB has value 1 if the patient has diabetes and 0 if not

In function F1,

$0.10 \leq a \leq 0.2$, more preferably $0.125 \leq a \leq 0.175$, in particular a= 0.148
$0.03 \leq \beta1 \leq 0.2$, more preferably $0.05 \leq \beta1 \leq 0.15$, in particular β1 = 0.09445
$-2.45 \leq \beta2 \leq -2.05$ , more preferably $-2.3 \leq \beta2 \leq -2.2$, in particular β2 = - 2.25926
$-2.4 \leq \beta3 \leq -2$, more preferably $-2.27 \leq \beta3 \leq -2.17$, in particular β3 = -2.22330
$-2.40 \leq \beta4 \leq -2$, more preferably $-2.30 \leq \beta4 \leq -2.18$, in particular β4 = -2.27153
$-20.6 \leq \beta5 \leq -20$, more preferably $-20.4 \leq \beta5 \leq -20.2$, in particular β5 = - 20.30707
$-20.3 \leq \beta6 \leq -19.7$, more preferably $-20.1 \leq \beta6 \leq -19.9$, in particular β6 = - 19.98760
$-2.5 \leq \beta7 \leq -1.9$, more preferably $-2.30 \leq \beta7 \leq -2.1$, in particular β7 = -2.18903
$-1.5 \leq \beta8 \leq -1$, more preferably $-1.35 \leq \beta8 \leq -1.15$, in particular β8 = -1.26744
$0.75 \leq \beta9 \leq 1.05$, more preferably $0.8 \leq \beta9 \leq 1.0$, in particular β9 = 0.89982

$0.5 \leq \beta10 \leq 0.9$, more preferably $0.65 \leq \beta10 \leq 0.75$, in particular $\beta10 = 0.72182$

$-0.8 \leq \beta11 \leq -0.4$, more preferably $-0.65 \leq \beta11 \leq -0.55$, in particular $\beta11 = -0.61001$

$0.45 \leq \gamma \leq 0.85$, more preferably $0.6 \leq \gamma \leq 0.7$, in particular $\gamma = 0.66405$

The values have been rounded to the third or fifth decimal.

In particular,

$$F1 = 1 - (1 - 0.148)^{\exp(f1)}$$

with f2 = 0.09445*tumt1B - 2.25926*tumt1C - 2.22330*tumt2A - 2.27153*tumt2B - 20.30707*tumt2C - 19.98760*tumt3 - 2.18903*tumt3A - 1.26744*tumt3B + 0.89982*URI1 + 0.72182*URI2 - 0.61001*DIAB + 0.66405.

This function has an AUROC of 0.658, therefore being of good prognosis quality. With a cut-off (reference end-value) of 0.042, the sensitivity is 0.493, specificity is 0.779, positive predictive value is 0.524 and negative predictive value is 0.757. This function with this cut-off is thus appropriate to identify the patients with low risk of having PSE.

It is to be noted that another cut-off can be determined with the physician, should there be a wish to particularly improve one or the other parameter (Sp, Se, PPV or NPV).

Another model was developed by adding the presence/absence and grade of gastro-intestinal toxicity. This made it possible to obtain a function F2:

$$F2 = 1 - (1 - a)^{\exp(f2)}$$

with f2 = $\beta1$*tumt1B + $\beta2$*tumt1C + $\beta3$*tumt2A + $\beta4$*tumt2B + $\beta5$*tumt2C + $\beta6$*tumt3 + $\beta7$*tumt3A + $\beta8$*tumt3B + $\beta9$*URI1 + $\beta10$*URI2 + $\beta11$*DIAB + $\beta12$*DIG1 + $\beta13$*DIG2 + $\gamma$

In this function f2

tumt1B has value 1 if the tumor T grade of the patient is 1B and 0 if not

tumt1C has value 1 if the tumor T grade of the patient is 1C and 0 if not

tumt2A has value 1 if the tumor T grade of the patient is 2A and 0 if not

tumt2B has value 1 if the tumor T grade of the patient is 2B and 0 if not

tumt2C has value 1 if the tumor T grade of the patient is 2C and 0 if not

tumt3 has value 1 if the tumor T grade of the patient is 3 and 0 if not

tumt3A has value 1 if the tumor T grade of the patient is 3A and 0 if not

tumt3B has value 1 if the tumor T grade of the patient is 3B and 0 if not

URI1 has value 1 if the urinary toxicity is of grade 1 and 0 if not

URI2 has value 1 if the urinary toxicity is of grade 2 and 0 if not.

DIAB has value 1 if the patient has diabetes and 0 if not

DIG1 has value 1 if the gastrointestinal toxicity is of grade 1 and 0 if not

DIG2 has value 1 if the gastrointestinal toxicity is of grade 1 and 0 if not.

In functions F2 and f2,

$0.10 \leq a \leq 0.2$, more preferably $0.125 \leq a \leq 0.175$, in particular a= 0.145

$0.03 \leq \beta1 \leq 0.1$, more preferably $0.04 \leq \beta1 \leq 0.08$, in particular $\beta1 = 0.06292$

$-2.5 \leq \beta2 \leq -2.2$, more preferably $-2.4 \leq \beta2 \leq -2.3$, in particular $\beta2 = -2.43992$

$-2.5 \leq \beta3 \leq -2.2$, more preferably $-2.4 \leq \beta3 \leq -2.3$, in particular $\beta3 = -2.34531$

$-2.5 \leq \beta4 \leq -2.2$, more preferably $-2.4 \leq \beta4 \leq -2.3$, in particular $\beta4 = -2.34962$

$-21 \leq \beta5 \leq -20.2$, more preferably $-20.8 \leq \beta5 \leq -20.4$, in particular $\beta5 = -20.60671$

$-20.3 \leq \beta6 \leq -19.7$, more preferably $-20.1 \leq \beta6 \leq -19.9$, in particular $\beta6 = -20.07638$

$-2.6 \leq \beta7 \leq -2.2$, more preferably $-2.45 \leq \beta7 \leq -2.35$, in particular $\beta7 = -2.40979$

$-1.5 \leq \beta8 \leq -1.1$, more preferably $-1.35 \leq \beta8 \leq -1.25$, in particular $\beta8 = -1.30754$

$0.75 \leq \beta9 \leq 1.05$, more preferably $0.8 \leq \beta9 \leq 1.0$, in particular $\beta9 = 0.88297$

$0.7 \leq \beta10 \leq 1$, more preferably $0.78 \leq \beta10 \leq 0.88$, in particular $\beta10 = 0.83043$

$-0.85 \leq \beta11 \leq -0.5$, more preferably $-0.72 \leq \beta11 \leq -0.62$, in particular $\beta11 = -0.67117$

$0.6 \leq \beta12 \leq 1$, more preferably $0.74 \leq \beta12 \leq 0.85$, in particular $\beta12 = 0.78775$

$1.0 \leq \beta13 \leq 1.3$, more preferably $1.1 \leq \beta13 \leq 1.2$, in particular $\beta13 = 1.15787$

$0.5 \leq \gamma \leq 0.9$, more preferably $0.65 \leq \gamma \leq 0.75$, in particular $\gamma = 0.70736$

The values have been rounded to the third or fifth decimal.

In particular,

$$F2 = 1 - (1 - 0.145)^{\exp(f2)}$$

with f2 = 0.06292*tumt1B - 2.43992*tumt1C - 2.34531*tumt2A - 2.34962*tumt2B - 20.60671*tumt2C - 20.07638*tumt3 - 2.40979*tumt3A - 1.30754*tumt3B + 0.88297*URI1 + 0.83043*URI2 - 0.67117*DIAB + 0.78775*DIG1 + 1.15787*DIG2 + 0.70736.

This function has an AUROC of 0.689, therefore being of good prognosis quality. With a cut-off (reference end-value) of 0.034, the sensitivity is 0.582, specificity is 0.713, positive predictive value is 0.500 and negative predictive value is 0.776. This function with this cut-off is thus appropriate to identify the patients with low risk of having PSE.

It is to be noted that another cut-off can be determined with the physician, should there be a wish to particularly improve one or the other parameter (Sp, Se, PPV or NPV).

Another model was developed by combining the tumor T stage, the presence and grade of some urinary disorders (in particular urinary irritation), the presence/absence and grade of gastro-intestinal toxicity, the tumor N stage and the presence/absence of lymph node dissection. This made it possible to obtain a function F3:

$$F3 = 1 - (1 - a)^{\exp(f3)}$$

with f3 = β1*tumt1B + β2*tumt1C + β3*tumt2A + β4*tumt2B + β5*tumt2C + β6*tumt3 + β7*tumt3A + β8*tumt3B + β9*URI0 + β10*DIG1 + β11*DIG2 + β12*tumn2 + β13*tumn3 + β14*LND + γ

In this function f2

tumt1B has value 1 if the tumor T grade of the patient is 1B and 0 if not
tumt1C has value 1 if the tumor T grade of the patient is 1C and 0 if not
tumt2A has value 1 if the tumor T grade of the patient is 2A and 0 if not
tumt2B has value 1 if the tumor T grade of the patient is 2B and 0 if not
tumt2C has value 1 if the tumor T grade of the patient is 2C and 0 if not
tumt3 has value 1 if the tumor T grade of the patient is 3 and 0 if not
tumt3A has value 1 if the tumor T grade of the patient is 3A and 0 if not
tumt3B has value 1 if the tumor T grade of the patient is 3B and 0 if not
URI0 has value 1 if the urinary toxicity is of grade 1 and 0 if not
DIG1 has value 1 if the gastrointestinal toxicity is of grade 1 and 0 if not
DIG2 has value 1 if the gastrointestinal toxicity is of grade 1 and 0 if not.
tumn2 has value 1 if the tumor N grade of the patient is 2 and 0 if not
tumn3 has value 1 if the tumor N grade of the patient is 3 and 0 if not
LND has value 1 if the patient has had lymph node dissection and 0 if not

In functions F3 and f3,

0.08 ≤ a ≤ 0.2, more preferably 0.1 ≤ a ≤ 0.15, in particular a= 0.124
0.42 ≤ β1 ≤ 0.72, more preferably 0.52 ≤ β1 ≤ 0.62, in particular β1 = 0.57485
-2.1 ≤ β2 ≤ -1.8 , more preferably -2 ≤ β2 ≤ -1.9, in particular β2 = -2.05310
-2.3 ≤ β3 ≤ -2, more preferably -2.2 ≤ β3 ≤ -2.1, in particular β3 = -2.16704
-2.15 ≤ β4 ≤ -1.85, more preferably -2.05 ≤ β4 ≤ -1.95, in particular β4 = - 2.00642
-20.25 ≤ β5 ≤ -19.75, more preferably -20.1 ≤ β5 ≤ -19.9, in particular β5 = - 20.02073
-20 ≤ β6 ≤ -19.5, more preferably -19.8 ≤ β6 ≤ -19.7, in particular β6 =-19.73967
-2.2 ≤ β7 ≤ -1.9, more preferably -2.1 ≤ β7 ≤ -2, in particular β7 = -2.04246
-1 ≤ β8 ≤ -0.7, more preferably -0.9≤ β8 ≤ -0.8, in particular β8 = -0.86255
0.85 ≤ β9 ≤ 1.15, more preferably 0.95≤ β9 ≤ 1.05, in particular β9 = 1.00952
1.03 ≤ β10 ≤ 1.33, more preferably 1.13 ≤ β10 ≤ 1.23, in particular β10 = 1.17941
0.28 ≤ β11 ≤ 0.58, more preferably 0.38 ≤ β11 ≤ 0.48, in particular β11 = 0.43277
1.35 ≤ β12 ≤ 1.65, more preferably 1.45 ≤ β12 ≤ 1.55, in particular β12 = 1.49829
-17.45 ≤ β13 ≤ -17.0, more preferably -17.3 ≤ β13 ≤ -17.1, in particular β13 =-17.21793
-1.35 ≤ β14 ≤ -1.05, more preferably -1.25 ≤ β14 ≤ -1.15, in particular β14 =-1.19765
0.25 ≤ γ ≤ 0.55 , more preferably 0.35 ≤ γ ≤ 0.45, in particular γ = 0.39655

The values have been rounded to the third or fifth decimal.

In particular,

$$F3 = 1 - (1 - 0.124)^{\exp(f3)}$$

with f3 = 0.57485*tumt1B - 2.05310*tumt1C - 2.16704*tumt2A - 2.00642*tumt2B - 20.02073*tumt2C - 19.73967*tumt3 - 2.04246*tumt3A - 0.86255*tumt3B + 1.00952*URI0 + 1.17941*DIG1 + 0.43277*DIG2 + 1.49829*tumn2 - 17.21793*tumn3 - 1.19765*LND + 0.39655.

This function has an AUROC of 0.707, therefore being of good prognosis quality. With a cut-off (reference end-value) of 0.043, the sensitivity is 0.578, specificity is 0.752, positive predictive value is 0.536 and negative predictive value is 0.782. This function with this cut-off is thus appropriate to identify the patients with low risk of having PSE.

It is to be noted that another cut-off can be determined with the physician, should there be a wish to particularly improve one or the other parameter (Sp, Se, PPV or NPV).

Other functions were developed combining the tumor T stage, the presence (and grade) of urinary toxicity, and other parameters (age, intake of anticoagulants, tumor N grade, presence of gastro-intestinal toxicity, level of PSA, concomitant hormonotherapy). Various combinations of these parameters were used together with tumor T stage and presence (and grade) of urinary toxicity.

The functions obtained presented AUROC comprised between 0.699 and 0.717, a number of them having an AUROC higher than 0.7. Negative Predictive Value varied to about 0.8 and up to 0.823. Since the goal of these functions is to determine whether a radiation treatment can be proposed and performed in a patient, such high NPVs show that these functions are particularly adapted.

This example shows that using the tumor T stage and presence (and grade) of urinary toxicity, and optionally other parameters in a mathematical function makes it possible to obtain a function that can be used for making prognosis of the occurrence of side effect after ionizing radiation treatment in patients with prostate cancer.

Other functions can be developed, using the methods disclosed above, and with other parameters added to the two herein disclosed.

## Claims

1. An *in vitro* method for assessing the risk of developing side effects after ionizing radiation treatment in a patient suffering from prostate cancer, comprising:

   a) determining the presence or absence, of urinary toxicity in the patient prior to application of ionizing radiation,
   b) determining the stage of the patient's tumor, preferably the tumor T stage
   c) optionally determining of at least one other clinical parameter, disease parameter or ionizing radiation treatment parameter from the patient, and
   d) combining a value associated with the presence of urinary toxicity determined in a), a value associated with the stage of the tumor in b), and a value association with the at least one other clinical parameter, disease parameter or ionizing radiation treatment parameter determined in c) in a mathematical function to obtain an end-value.

2. The *in vitro* method according to claim 1, wherein a) also comprises the determination of the grade of the urinary toxicity if such is present in the patient.

3. The *in vitro* method according to claim 1 or 2, wherein c) comprises the determination of at least another clinical marker, selected from the group consisting in the presence or absence of concomitant and/or adjuvant hormonotherapy, the tobacco smoking habit, the presence or absence of gastrointestinal toxicity, the dosage of PSA (Prostate Specific Antigen), presence of at least one comorbidity, the presence or absence of lymph node dissection, tumor N stage and the age of the patient, and wherein a value associated with said at least another clinical marker is combined in the function of c).

4. The *in vitro* method according to claim 3, wherein the comorbidity is selected from the group consisting of presence or diabetes and high blood pressure associated with intake of anticoagulant drugs.

5. The *in vitro* method according to anyone of claims 1 to 4, wherein the end value is compared with a reference end-value, thereby determining if the subject present a high or low risk to develop radiation-induced side-effects .

6. The *in vitro* method according to any one of claims 1 to 5, wherein said mathematical function is a multivariate analysis using binary logistic regression, a multiple linear regression or any time dependent regression.

7. The *in vitro* method according to any one of claims 1 to 6, wherein said mathematical function is a COX proportional hazard regression model.

8. The *in vitro* method according to anyone of claims 1 to 7, wherein said radiation-induced side effect occurs within 24 months after the end of the ionizing radiation.

9. The *in vitro* method according to anyone of claims 1 to 8 wherein said radiation-induced side effect is selected from urinary toxicities, gastrointestinal toxicities, sexual toxicities and pelvic neuropathy.

10. The *in vitro* method according to anyone of claims 1 to 9, wherein said method is computerized.

11. A microprocessor comprising a software for implementing the *in vitro* method according to anyone of claims 1 to 10.

12. A method for treating a patient having prostate cancer, comprising:

a) performing the method according to any one of claims 1 to 10, and
b) treating the patient with ionizing radiation, if the end result indicates that the subject has no or a low risk of developing side effects after ionizing radiation,
c) providing another treatment or adapting the ionizing radiation treatment if the patient doesn't have no or a low risk of developing side effects after ionizing radiation.

13. A method for obtaining a mathematical function that can be used in an *in vitro* non-invasive prognosis test for assessing the risk of developing side effects after ionizing radiation in a subject suffering from prostate cancer, comprising:

g) classifying subjects of a cohort of patients into different groups according to the presence of side effects after ionizing radiation treatment
h) determining the presence or absence of urinary toxicity prior to ionizing radiation treatment,
i) determining the stage of the patient's tumor, preferably the tumor T stage
j) optionally determining the presence of at least one other marker selected from the group consisting in the presence or absence of concomitant and/or adjuvant hormonotherapy, the tobacco smoking habit, the presence or absence of gastrointestinal toxicity, the dosage of PSA (Prostate Specific Antigen), the presence of at least one comorbidity (in particular diabetes), the presence or absence of lymph node dissection, tumor N stage and the age of the patient, before ionizing radiation treatment
k) identifying by unidimensional analysis, the markers measured in step d) for which the measured value differs significantly between the group of subjects in which side effects after ionizing radiation have developed and the group of subjects in which side effects after ionizing radiation have not occurred, thereby obtaining independent factors
l) obtaining the mathematical function by combination of the factor measured in b), the marker determined in c) and the identified independent factors identified in e).

| | Europäisches<br>Patentamt<br>European<br>Patent Office<br>Office européen<br>des brevets | **EUROPEAN SEARCH REPORT** | Application Number<br>EP 19 30 5263 |
|---|---|---|---|

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| A | WOUTER SCHAAKE ET AL: "Development of a prediction model for late urinary incontinence, hematuria, pain and voiding frequency among irradiated prostate cancer patients",<br>PLOS ONE,<br>vol. 13, no. 7, 17 July 2018 (2018-07-17),<br>page e0197757, XP55613495,<br>DOI: 10.1371/journal.pone.0197757<br>* the whole document *<br>* In particular: Title; Abstract;<br>Materials and methods. *<br>----- | 1-13 | INV.<br>G01N33/50 |
| A | VIVIANA CARILLO ET AL: "Relationships between bladder dose-volume/surface histograms and acute urinary toxicity after radiotherapy for prostate cancer",<br>RADIOTHERAPY AND ONCOLOGY,<br>vol. 111, no. 1, 1 April 2014 (2014-04-01)<br>, pages 100-105, XP55613287,<br>Ireland<br>ISSN: 0167-8140, DOI:<br>10.1016/j.radonc.2014.02.006<br>* the whole document *<br>* In particular: Title; Abstract;<br>Materials and methods. *<br>-----<br>-/-- | 1-13 | TECHNICAL FIELDS<br>SEARCHED (IPC)<br><br>G01N |

The present search report has been drawn up for all claims

| Place of search | Date of completion of the search | Examiner |
|---|---|---|
| The Hague | 19 August 2019 | C.F. Angioni |

EPO FORM 1503 03.82 (P04C01)

page 1 of 2

Europäisches
Patentamt

European
Patent Office

Office européen
des brevets

## EUROPEAN SEARCH REPORT

Application Number

EP 19 30 5263

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JANA VRANOVA ET AL: "The evolution of rectal and urinary toxicity and immune response in prostate cancer patients treated with two three-dimensional conformal radiotherapy techniques", RADIATION ONCOLOGY, BIOMED CENTRAL LTD, LO, vol. 6, no. 1, 27 July 2011 (2011-07-27), page 87, XP021105925, ISSN: 1748-717X, DOI: 10.1186/1748-717X-6-87 * the whole document * * In particular: Title; Abstract; Methods. * | 1-13 | |

TECHNICAL FIELDS
SEARCHED     (IPC)

The present search report has been drawn up for all claims

| Place of search | Date of completion of the search | Examiner |
|---|---|---|
| The Hague | 19 August 2019 | C.F. Angioni |

EPO FORM 1503 03.82 (P04C01)

page 2 of 2

# EP 3 705 891 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016008629 A

- WO 2018041960 A

**Non-patent literature cited in the description**

- **FORO et al.** *Int J Radiat Oncol Biol Phys.,* 01 April 2014, vol. 88 (5), 1057-63
- **DE LANGHE et al.** *Radiotherapy and Oncology,* 2013, vol. 106, S350

- **D'AMICO et al.** *JAMA,* 1998, vol. 280 (11), 969-74
- **AZRIA et al.** *Front Oncol.,* 27 April 2017, vol. 7, 83